# EUROPEAN PATENT APPLICATION

(11) **EP 2 835 103 A1**
(43) Date of publication of application: **11.02.2015**
(21) Application number: 14178993.3
(22) Date of filing: 29.07.2014
(51) Int. Cl.: A61B 6/02, A61B 6/00, G03B 35/04

(54) **Imaging system and method for portable stereoscopic tomography**

(30) Priority: 07.08.2013 US 201361862975 P; 26.02.2014 US 201414190447
(71) Applicant: Carestream Health, Inc., Rochester, NY 14608 (US)
(72) Inventor: Foos, David H., Rochester, New York 14607 (US); Simon, Richard A., 14626, New York Rochester, (US); Wang, Xiaohui, Pittsford, New York 14534 (US)
(74) Representative: Emde, Eric

(57) **Abstract**

A portable imaging apparatus for obtaining stereoscopic radiography images has a transport frame that has a support structure and an x-ray source array that is coupled to the support structure and that has three or more x-ray sources, each x-ray source separately energizable to emit radiation toward a subject. There is at least one x-ray detector adapted to acquire digital image data according to radiation received from the x-ray sources of the x-ray source array. An image processor is in signal communication with the at least one x-ray detector and adapted to generate, from the acquired digital image data, a right-eye digital image and a left-eye digital image of the subject. A display apparatus is in signal communication with the image processor and is adapted to display the right-eye and left-eye digital images of the subject to a viewer.

## Description

### TECHNICAL FIELD

The disclosure relates generally to the field of radiography and more particularly to apparatus and methods for generating and displaying stereoscopic images from a radiography system.

### BACKGROUND

Interventional radiology (IR) relates to a range of surgical procedures that are performed with the aid of fluoroscopic imaging equipment. Fluoroscopy provides motion x-ray based imaging, often used in concert with an iodinated contrast agent. For example, fluoroscopy can help to guide a catheter through the veins of a patient in order to reach a location in the patient's body that requires some form of treatment, e.g., the site of a tumor or abscess. Once a catheter is appropriately positioned, a treatment delivery device can be inserted through the catheter to the site of the abnormality and a treatment applied. Such treatment may involve embolization to cut off the blood supply that feeds a tumor, or to ablate a tumor using thermal methods, or, in the case of an abscess, to drain infectious material.

The fluoroscopy-guided process of catheter placement requires considerable skill and often entails an amount of risk. Guidance procedures may be time consuming and can be difficult to execute, since the ability to visualize a catheter tip as it progressively advances through complex venous structures is inhibited by problems such as poor subject contrast and dose constraints.

State of the art interventional radiology procedures have been performed using expensive C-arm systems equipped with high frame rate flat panel detectors that provide two-dimensional (2-D) images. These apparatus are conventionally coupled with power contrast injection systems. However, the real time motion images that are viewed on heads-up monitors by interventional radiologists (as they carefully advance catheters into position) are limited in value, constrained in large part by their two dimensional nature. Thus, the motion images that are obtained from this effort often provide little or no depth perception information. This is true even though the procedure itself is 3-dimensional.

Tomography (also referred to as x-ray computed tomography or computed tomography (CT)) is a well known medical digital imaging method available using computer processing to acquire and combine image data from multiple angles. Digital image processing is used to generate a three-dimensional image of the inside of an object from a series/collection of two-dimensional x-ray images taken around a single axis of rotation. In CT, a source/detector makes a complete 360-degree rotation about the subject obtaining a complete volume of data from which images may be reconstructed. The volume of data produced by the CT system is manipulated to generate body structures. The images can be generated in the axial or transverse plane (e.g., perpendicular to the long axis of the body), or reformatted in various planes, or volumetric three-dimensional representations.

Tomosynthesis combines digital image capture and processing with source/detector motion used in tomography. While there are some similarities to CT, some view tomosynthesis as a separate technique. As noted above, in CT, the source/detector makes a complete 360-degree rotation about the subject obtaining a complete set of data from which images may be reconstructed. By contrast, digital tomosynthesis uses a small rotation angle (e.g., 30 degrees) with a small number of discrete slices/exposures (e.g., 10). This set of data, incomplete with regard to full volume image information, is digitally processed to yield images similar to tomography with a limited depth of field. Since the image is digitally processed, a series of slices at different depths and with different thicknesses can be reconstructed from the same acquisition, thereby saving time and reducing radiation exposure.

Because the tomosynthesis data acquired is incomplete in terms of the full three dimensions of data content, tomosynthesis does not offer the narrow slice widths that CT offers. However, tomosynthesis provides a measure of depth detail that is not otherwise available with conventional 2-D radiography. Moreover, the limited depth detail information can be of value for providing a measure of stereoscopic display to the viewer. Stereoscopic display provides improved visualization over conventional 2-D image presentation.

Thus, it can be seen that there is a need for a portable imaging apparatus that is capable of providing stereoscopic imaging in an intensive care unit (ICU), emergency room (ER), or other environment.

### SUMMARY

An object of the present disclosure is to address the need for a portable tomographic apparatus that provides stereoscopic images of patient anatomy. A related object of the present disclosure is to provide apparatus and methods for volume imaging with suitable depth display for the practitioner. The mobility of the imaging apparatus of the present invention allows its use in interventional radiology, fluoroscopy, and surgery, for example. Mobile aspects of the present disclosure take advantage of the digital radiography (DR) detector that can be positioned independently from the radiation source. Various configurations can be used, taking advantage of array arrangements for radiography sources.

These objects are given only by way of illustrative example, and such objects may be exemplary of one or more embodiments of the invention. Other desirable objectives and advantages inherently achieved by the disclosed apparatus may occur or become apparent to those skilled in the art. The invention is defined by the appended claims.

According to one aspect of the disclosure, there is provided a portable imaging apparatus for obtaining stereoscopic radiography images, the apparatus comprising: a) a mobile transport frame that has a support structure; b) an x-ray source array that is coupled to the support structure and that has three or more x-ray sources, each x-ray source separately energizable to emit radiation toward a subject; c) at least one x-ray detector adapted to acquire digital image data according to radiation received from the x-ray sources of the x-ray source array; d) an image processor in signal communication with the at least one x-ray detector and adapted to generate, from the acquired digital image data, a right-eye digital image and a left-eye digital image of the subject; and e) a display apparatus that is in signal communication with the image processor and that is adapted to display the right-eye and left-eye digital images of the subject to a viewer.

### BRIEF DESCRIPTION OF THE DRAWINGS

The foregoing and other objects, features, and advantages of the invention will be apparent from the following more particular description of the embodiments of the invention, as illustrated in the accompanying drawings. The elements of the drawings are not necessarily to scale relative to each other.
FIG. 1 is a diagram that shows a perspective view of a mobile radiography unit with a second display according to one embodiment of the application.
FIG. 2 is a diagram that shows a perspective view of a mobile radiography unit of FIG. 1 positioned for travel.
FIG. 3 is a diagram that shows an exemplary embodiment of a display/monitor as a second display mounted to a boom assembly of a mobile radiography unit according to the application.
FIG. 4 is a schematic diagram that shows a stereoscopic fluoroscopy apparatus according to an embodiment of the present disclosure.
FIG. 5 is a diagram that shows a perspective view of a mobile radiography unit that can provide a tomosynthesis capability according to embodiments of the present disclosure.
FIG. 6 is a diagram that shows x-ray source assemblies for exemplary mobile radiographic imaging systems including an x-ray source array embodiment that can include first and second radiographic x-ray sources and additional x-ray sources according to the application.
FIG. 7 is a diagram that shows alternate x-ray source assemblies for exemplary mobile radiographic imaging systems including an x-ray source array embodiment that can include first and second radiographic x-ray sources and additional x-ray sources according to the application.
FIG. 8A is a perspective view showing an arrangement of radiation sources for imaging according to an embodiment of the present disclosure.
FIG. 8B is a perspective view from above showing an arrangement of radiation sources for imaging according to an embodiment of the present disclosure.
FIG. 8C is a perspective view from below showing the arrangement of radiation sources shown in FIGS. 8A and 8B for imaging according to an embodiment of the present disclosure.
FIG. 8D is a perspective view showing an arrangement of radiation sources for imaging along with collimators for the source array according to an embodiment of the present disclosure.
FIG. 9A is a schematic view that shows acquisition of one member of the stereoscopic image pair using an array of sources.
FIG. 9B is a schematic view that shows acquisition of the complementary member of the stereoscopic image pair to that of FIG. 9A using an array of sources.
FIG. 9C is a schematic view that shows acquisition of one member of the stereoscopic image pair using an array of sources, wherein the view angle is from one side of the patient.
FIG. 9D is a schematic view that shows acquisition of the complementary member of the stereoscopic image pair to that of FIG. 9C using an array of sources.
FIG. 10A is a schematic view that shows acquisition of one member of the stereoscopic image pair using an array of sources.
FIG. 10B is a schematic view that shows acquisition of the complementary member of the stereoscopic image pair to that of FIG. 10A using an array of sources.
FIG. 10C is a schematic view that shows acquisition of the complementary member of the stereoscopic image pair to that of FIG. 10B using an array of sources.
FIG. 10D is a schematic view that shows acquisition of the complementary member of the stereoscopic image pair to that of FIG. 10C using an array of sources.
FIG. 11A is a top view showing the array of x-ray sources.
FIG. 11B is a side view showing exemplary emitted beam patterns from the array of x-ray sources.
FIG. 12A is a schematic view that shows the collimated beam intersection patterns at different distances from the source.
FIG. 12B is a perspective view that shows a shield and different types of radiation sources for combined tomosynthesis and general radiology.
FIG. 13 is a flow chart that shows an exemplary method of operating exemplary mobile radiographic imaging systems for acquiring projections images and generating the reconstruction of three-dimensional tomosynthesis images.
FIG. 14 shows simulations of exemplary projection images obtained using x-rays from each source position.
FIG. 15 shows a tomosynthesis reconstruction image according to an embodiment of the present disclosure.
FIG. 16A is a perspective view that shows a pair of stereoscopic viewing glasses as one type of wearable viewing apparatus that use synchronized shutters or other mechanism for differentiating right-eye and left-eye images of the stereo pair.
FIG. 16B shows a pair of wearable viewing apparatus having orthogonal polarizers.
FIG. 16C shows a pair of wearable viewing apparatus having spectral filters.
FIG. 17 is a top view that shows a practitioner or other observer wearing a head-mounted device.
FIG. 18A is a schematic view showing the head-mounted device used for standard viewing of the patient.
FIG. 18B is a schematic view that shows viewing a computer-generated image using the head-mounted device used for stereoscopic display.
FIG. 18C is a schematic view that shows the head-mounted device generating an image of operator interface controls.
FIG. 18D is a schematic view that shows the head-mounted device generating a combined image of operator interface controls superimposed on stereoscopic image content.
FIG. 18E is a schematic view that shows gestural interpretation used for command entry according to an embodiment of the present disclosure.
FIG. 18F is a schematic view that shows gestural interpretation used for command entry for computer-generated display adjustment according to an embodiment of the present disclosure.
FIG. 19A is a schematic view that shows an operator interface screen for selecting specific x-ray sources from an array of sources.
FIG. 19B is a schematic view that shows an operator interface screen and manual controls for selecting specific x-ray sources from an array of sources.
FIG. 19C is a schematic view that shows gestural interpretation used for command entry for selecting x-ray sources from an array of sources according to an embodiment of the present disclosure.
FIG. 19D is a schematic view that shows gestural interpretation used for command entry for selecting x-ray sources from an array of sources according to an embodiment of the present disclosure.

### DETAILED DESCRIPTION OF THE EMBODIMENTS

This application claims the benefit of U.S. Provisional Application Serial No. 61/862,975 filed on August 7, 2013, and U.S. Serial No. 14/190,447 filed on February 26, 2014, in the names of Xiaohui Wang, Richard A. Simon, and David H. Foos; both incorporated herein in their entirety.

The following is a detailed description of the preferred embodiments, reference being made to the drawings in which the same reference numerals identify the same elements of structure in each of the several figures.

Where they are used herein, the terms "first", "second", and so on, do not necessarily denote any ordinal, sequential, or priority relation, but are simply used to more clearly distinguish one element or set of elements from another, unless specified otherwise.

In the context of the present disclosure, the terms "viewer", "operator", "viewing practitioner", "observer", and "user" are considered to be equivalent and refer to the viewing practitioner or other person who views and manipulates an x-ray image on a display monitor or other viewing apparatus.

As used herein, the term "energizable" relates to a device or set of components that perform an indicated function upon receiving power and, optionally, upon receiving an enabling signal.

The term "actuable" has its conventional meaning, relating to a device or component that is capable of effecting an action in response to a stimulus, such as in response to an electrical signal, for example.

In the following disclosure, the phrase "left-eye image" denotes the image formed by a display apparatus and intended for viewing by the left eye of the viewer. Likewise, the phrase "right-eye image" refers to the complementary image that is intended for viewing from the right eye of the viewer. The term "stereo pair", "stereo image pair", or simply "stereo image" denotes the combination of right-eye image and corresponding complementary left-eye image for a stereoscopic view. A stereo pair can be hyperstereoscopic where there is an abnormally large separation distance between the angular views for the complementary left- and right-eye images, relative to the pupil-to-pupil distance of an average viewer. A stereo pair can alternately be hypostereoscopic where there is an abnormally small separation distance between the angular views for left- and right-eye images. For stereoscopic viewing, the terms "stereoscopic display apparatus" and "stereoscopic viewing apparatus" can be considered to provide equivalent functions, forming separate left- and right-eye images of a stereo pair for stereoscopic image presentation.

In the context of the present invention, the term "spectral range" or "spectral band" refers to a single wavelength or to a relatively narrow range of wavelengths of no more than about 40 nm. As is known to those skilled in the stereoscopic visualization arts, spectral stereo vision separation schemes project left- and right-eye images at different wavelengths for each primary color (Red, Green, or Blue, conventionally referred to as R, G, or B) and use filter elements to separate the left- and right-eye image content for each color.

Reference is made to an article by Je Hwang Ryu, Jung Su Kang, and Kyu Chang Park, entitled "Carbon Nanotube Electron Emitter for X-ray Imaging" in Materials, 2012, 5, 2353-2359.

Reference is made to U.S. Patent 8,172,633 (Park), filed April 4, 2007; U.S. Publication No. 2011/0003109 (Slinker), filed July 1, 2009; and U.S. Patent 7,505,562 (Dinca), filed April 19, 2007.

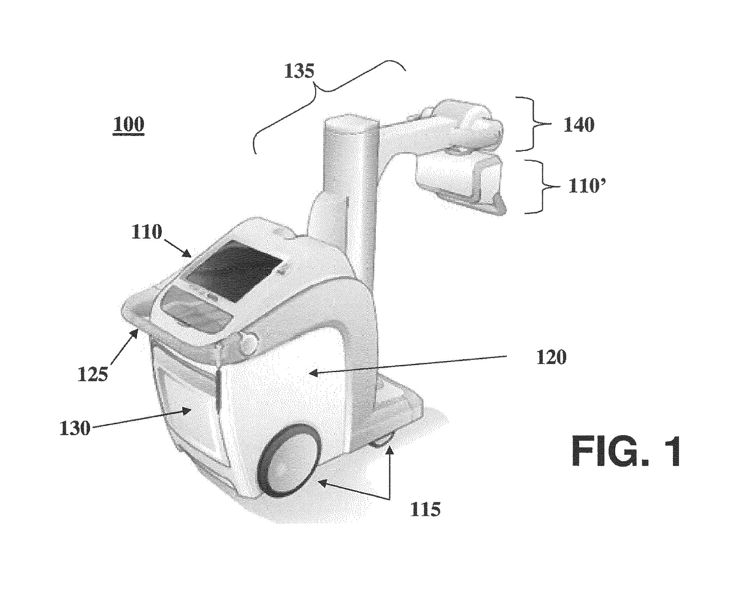

FIG. 1 is a diagram that shows a perspective view of a mobile radiography unit that can use one or more portable radiographic detectors or flat panel detectors adapted to acquire digital image data according to radiation received from the x-ray sources according to embodiments of the application. The exemplary mobile x-ray or radiographic apparatus of FIG. 1 can be employed for digital radiography (DR) and/or tomosynthesis. As shown in FIG. 1, a mobile radiography apparatus 100 can include a moveable transport frame 120 that includes a first display 110 and an optional second display 110' to display relevant information such as obtained images and related data. As shown in FIG. 1, the second display 110' can be pivotably mounted adjacent to the x-ray source 140 to be viewable/touchable from a 360 degree area.

The displays 110, 110' can implement or control (e.g., touch screens) functions such as generating, storing, transmitting, modifying, and printing of an obtained image(s) and can include an integral or separate control panel (not shown) to assist in implementing functions such as generating, storing, transmitting, modifying, and printing of an obtained image(s). One or more of displays 110, 110' can be separable from the apparatus 100 frame.

For mobility, the mobile radiographic apparatus 100 can have one or more wheels 115 and one or more handle grips 125, typically provided at waist-level, arm- level, or hand-level, that help to guide the mobile radiographic apparatus 100 to its intended location. A self-contained battery pack (e.g., rechargeable) can provide source power, which can reduce or eliminate the need for operation near a power outlet. Further, the self-contained battery pack can provide for motorized transport.

For storage, the mobile radiographic apparatus 100 can include an area/holder for holding/storing one or more digital radiographic (DR) detectors or computed radiography cassettes. The area/holder can be storage area 130 (e.g., disposed on the frame 120) configured to removably retain at least one digital radiography (DR) detector. The storage area 130 can be configured to hold a plurality of detectors and can also be configured to hold one size or multiple sizes of DR detectors and/or batteries therefore.

Mounted to frame 120 is a support member 135 that supports one or more x-ray sources 140, also called an x-ray tube, tube head, or generator that can be mounted to the support member 135. In the embodiment shown in FIG. 1, the support member 135 can extend vertically (e.g., column) and/or horizontally. Support member 135 can include a second section that extends outward a fixed/variable distance from a first section where the second section is configured to ride vertically up and down the first section to the desired height for obtaining the image. In addition, the support member is rotatably attached to the moveable frame 120. In another embodiment, the tube head or x-ray source 140 can be rotatably coupled to the support member 135. In another exemplary embodiment, an articulated member of the support member that bends at a joint mechanism can allow movement of the x-ray source 140 over a range of vertical and horizontal positions. Height settings for the x-ray source 140 can range from low height for imaging feet and lower extremities to shoulder height and above for imaging the upper body portions of patients in various positions.

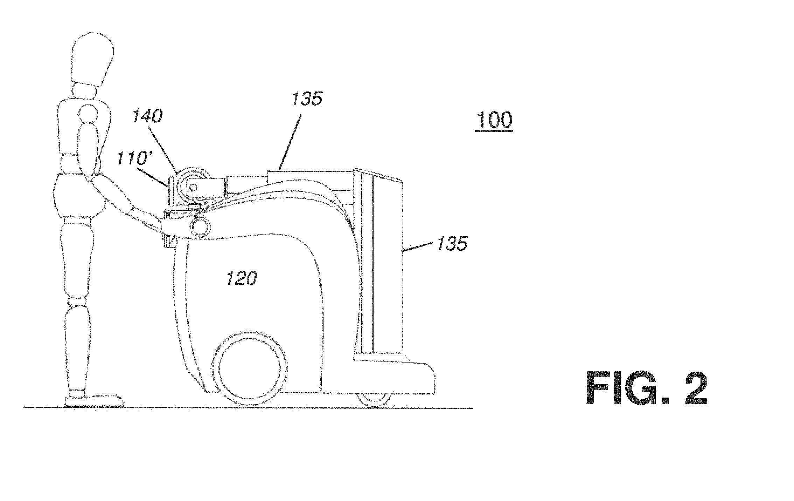

As shown in FIG. 2, for ease during transport of the mobile radiographic apparatus 100, the support member 135 and x-ray source 140 can be arranged close to frame 120. As shown in FIG. 2, the second display 110' can be in a viewable position (e.g., operable) during transport of the mobile radiographic apparatus 100. When the mobile radiographic apparatus 100 is to be used, the support member 135 and x-ray source 140 can be extended from the frame 120 for proper positioning (e.g., by the operator, a user, or x-ray technician) and the second display 110' moved to viewable position such as shown in FIG. 1.

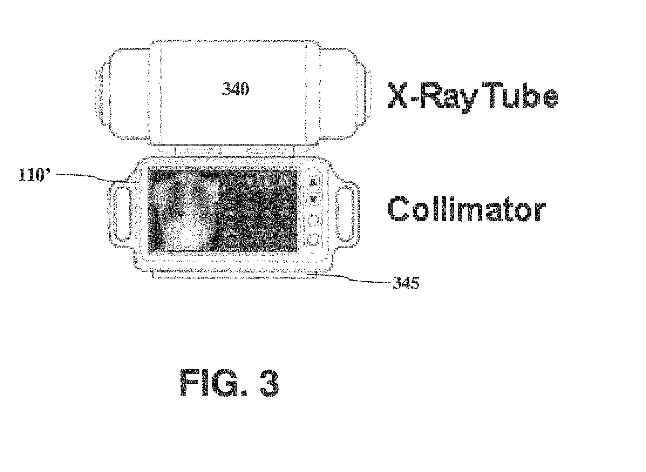

FIG. 3 is a diagram that shows an exemplary embodiment of a display/monitor as a second display mounted to a boom assembly of a mobile radiography unit according to the application. As shown in FIG. 3, the second display 110' can be mounted to a collimator 345 of an x-ray source 340 of a support member 135 of a mobile radiography unit. In one embodiment, the collimator 345 can be rotatably mounted to the x-ray source 340 so that the collimator 345 (e.g., second display 110') can swivel at least 90 degrees, at least 180 degrees or 360 degrees plus. As shown in FIG. 3, the second display 110' is coupled to a plurality of handles for ease of positioning. Alternatively, the second display 110' can be mounted to (e.g., rotatably) an x-ray source 340 above a collimator 345 of a boom assembly of a mobile radiography unit.

Applicants have developed a system and method to capture and display real-time x-ray motion sequences in stereo, and therefore allow interventional radiologists to perform fluoroscopy procedures while visualizing anatomy with a sense of depth. With improved visualization, IR procedures can be performed in less time, potentially with lower radiation burden to patients and potentially using smaller concentrations of contrast agent.

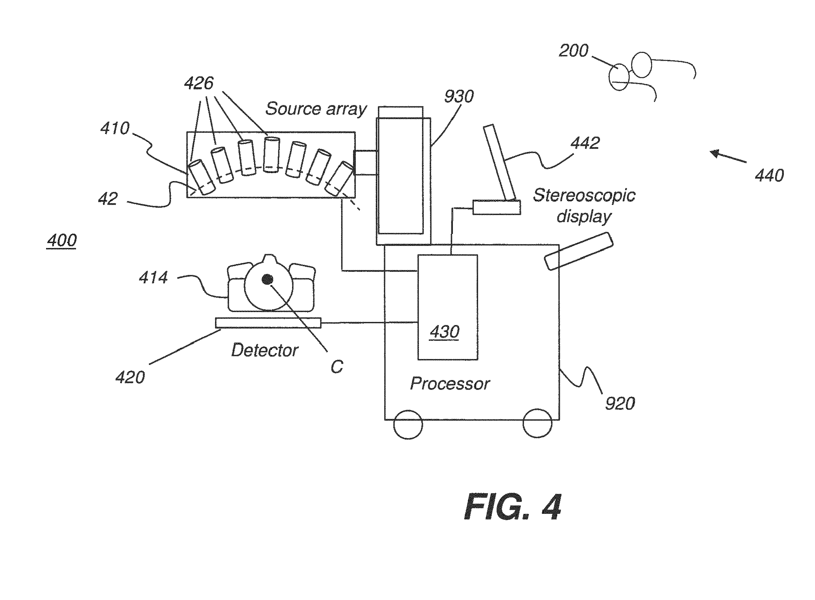

As shown in schematic form in FIG. 4, a portable or mobile stereoscopic fluoroscopy apparatus 400 for obtaining images of a patient 414 has an array of x-ray sources 410; an x-ray detector 420 that can be separable from a support column 930 and is adapted to capture a pair of digital image exposure sequences; an image processor 430, such as a computer server or workstation, for processing the pair of digital images and adapted to generate a stereo image pair; and a wearable stereoscopic viewing apparatus 440 with a display 442 that is in signal communication with processor 430 and is adapted to display the stereo image. Viewing glasses 200 are optionally worn as part of stereoscopic viewing apparatus 440 for viewing the stereoscopic image. A mobile transport frame 920 has column 930 that serves as a support structure for extending array 410 toward patient 414 or other subject to be imaged. X-ray source array 410 is then stationary during imaging. The array of x-ray sources 410 in FIG. 4 can have three or more x-ray sources 426 and is geometrically arcuate in a plane perpendicular to the image plane in the embodiment that is shown. Considered geometrically, the arc center, shown as C in FIG. 4 can be generally within the patient or within a meter of the patient. An arc 42 is represented in a dashed line.

This array 410 could alternately be designed so that sources 426 are arranged to be geometrically arcuate in a plane parallel to the image plane, or can have some other overall shape. Detector 420 is positionable independent of source array 410. According to an embodiment of the present disclosure, as shown in FIG. 4, transport frame 920 also houses processor 430 for stereoscopic image processing and presentation.

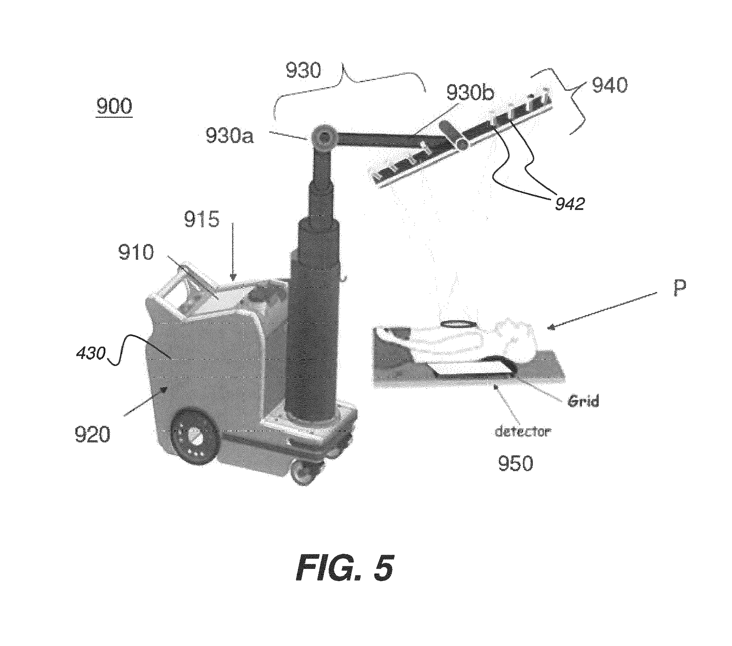

FIG. 5 is a diagram that shows a perspective view of a mobile radiography apparatus 900 that can provide a stereoscopic tomosynthesis imaging capability according to embodiments of the application. In one embodiment, a mobile radiography apparatus 900 can operate as a tomosynthesis or fluoroscopy system, or a system adapted for other volume imaging modes. As shown in FIGS. 4 and 5, an embodiment of a mobile radiography apparatus 900 can include a movable transport frame 920. Mounted to the moveable transport frame 920 can be a support column 930 that supports an x-ray source array 940. As shown in FIG. 5, support column 930 can include a second section 930b that extends outward a fixed/variable distance from a first section 930a, where the second section 930b is configured to move (e.g., ride vertically) up and down the first section 930a to the desired height for obtaining the projection images. The system also includes a digital x-ray detector 950 that is in signal communication with system controller 915. Signal communication is provided either wirelessly or in wired or tethered form, so that detector 950 is connected to system controller 915 contained inside the moveable transport frame 920. Detector 950 is positionable independent of x-ray source array 940 components. The system controller 915 can implement and/or control the functions of the mobile radiographic/tomosynthesis system 900 (e.g., functions provided through a console or control displays 110, 110' in FIG. 1). The system controller 915 can be provided though one or more of a conventional general purpose processor, digital computer, microprocessor, RISC processor, signal processor, CPU, arithmetic logic unit (ALU), video digital signal processor (VDSP), dedicated processor, and/or similar computational machines, programmed for a range of positioning and imaging functions according to the teachings of the application, as will be apparent to those skilled in the radiography imaging art.

In certain exemplary embodiments, mobile radiography apparatus 900 can provide a tomosynthesis capability. A moveable mounted x-ray source array 940 can, in addition, be supplied with a plurality of multiple individually controlled x-ray sources 942, such as more than three sources 942, to provide a distributed x-ray source array. FIG. 5 shows an embodiment of a mobile tomosynthesis system where multiple individually controlled x-ray sources 942 in a linear arrangement or pattern provide distributed x-ray sources in an array. As shown in FIG. 5, x-ray source array 940 can optionally include a plurality of distributed x-ray power sources 942 where at least one central source of the distributed x-ray power sources has full (e.g., standard) x-ray power. The central source, for example, can have a wide range of kVp settings, such as from 50 kVp to 150 kVp, and high maximum mA output, such as from 10 mA to 400 mA, in order to accommodate many different exam types for general radiography. The x-ray sources can include at least a first x-ray source that generates radiation exceeding 60 kVp and at least a second x-ray source that generates radiation below about 40 kVp.

The distributed sources that form the array can be disposed in a prescribed spatial relationship. The distributed sources can include lower power x-ray sources, which means a narrow range of kVp settings, such as from 60 kVp to 120 kVp for example, or such as from 30kVp to 130 kVp, and lower maximum mA output, such as from 1 mA to 100 mA. X-ray source array 940 can use one or more collimators to form beams that are directed towards the detector 950 and/or patient P. The x-ray source array 940 may also include positioning mechanisms, such as motors, that allow for directing the beam more accurately towards the detector 950 and/or patient P. The moveable transport frame 920 can include a first display 910, which can be part of a control console to control at least the x-ray source array 940. Further, the system controller 915 can coordinate operations of the x-ray source array 940, detector 950, and moveable transport frame 920 (e.g., via operator actions using the first display 910). The system controller 915 can control operations of the x-ray source array, which may include collimator settings, positioning devices and triggering of image acquisition by emission of x-rays from the sources. For example, the system controller 915 can control x-ray emission for CT imaging procedures and/or for general radiography imaging procedures. The system controller 915 also can control operations of detector 950, which may include triggering of image acquisition process and transmission of the acquired images back to the controller. In addition, the system controller 915 can control the movement of the transport frame 920.

### Array of x-ray sources

The x-ray sources can be, for example, a distributed array of field-emission based X-ray sources, such as sources having carbon nanotube (CNT) cathodes. The X-ray sources are stationary or relatively fixed in position with respect to each other within the array; the array itself moves as a single unit. This type of x-ray source is capable of rapid on/off switching on the order of microseconds. Other suitable x-ray sources can include paired pulsed conventional fluoro-capable thermionic sources that are spatially separated. These options provide sufficient x-ray fluence with short exposure times and simultaneously allow exposure sequences without overheating.

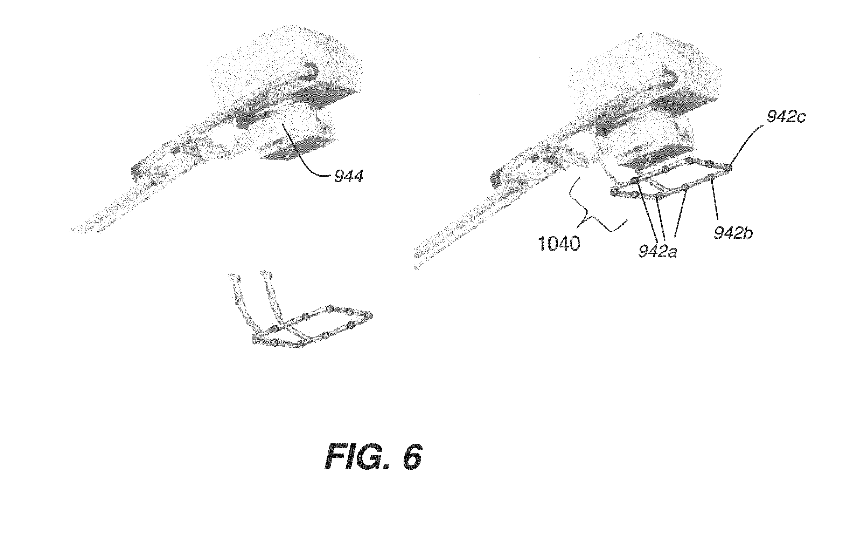

The diagram of FIG. 6 shows an x-ray source array 1040 of a mobile radiographic imaging system that includes a first radiographic x-ray source 944 and collimator, and a second, third, and additional x-ray sources 942a, 942b, 942c, and so on, that can be individually adjusted (e.g., collimated) and either permanently attached or attached (e.g., detachable) when needed. As shown in FIG. 6, according to an embodiment of the present disclosure, the first radiographic x-ray source 944 can be a central one of the distributed sources. Alternatively, the first radiographic x-ray source is positioned at a center of the second array of distributed sources. As shown in FIG. 6, the first radiographic x-ray source 944 can be a mobile/portable x-ray source/tube and can be a different type of x-ray source from the second distributed array of lower power carbon-nanotube x-ray sources. Other types of standard radiography and distributed array sources can be used. Detachable sources or the full detachable array 410 can be separately mounted from the support structure of stereoscopic fluoroscopy apparatus 400 (FIG. 4) or supported at one or more positions around the patient.

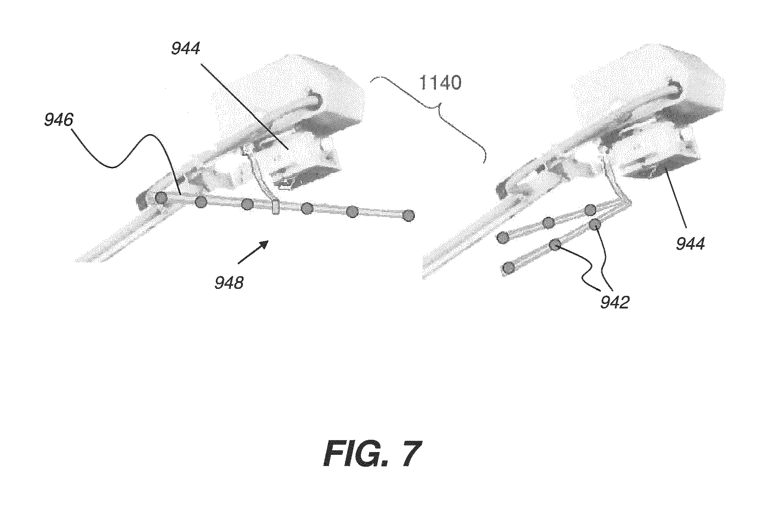

The diagram of FIG. 7 shows an alternate embodiment of an x-ray source array 1140 of a mobile radiographic imaging system. The linear x-ray source array 1140 can include a directed first radiographic x-ray source 944 and a directed second x-ray source array 948 comprising a distributed source attachment (e.g., linear) that can be either permanently attached or attached (detachable) when needed. As shown in FIG. 7, the first radiographic x-ray source can be positioned at a center of the array of distributed sources. In one embodiment, the first radiographic x-ray source can be a central member of the array 948 of distributed sources. In one embodiment, the plurality of distributed x-ray sources 942 can be mounted along a support 946. In one embodiment, the plurality of distributed x-ray sources 942 can have a prescribed spatial relationship, where the prescribed spatial relationship is one or more linear tracks, 2-D tracks, curves, polygons, rectangles or 3D paths. In one embodiment, collimated distributed sources can be on a curved support to maintain a single distance from a corresponding point on a detector. Exemplary distributed source attachment can have a first position for use and a second position for storage (e.g., folded as shown in FIG. 7) when not used.

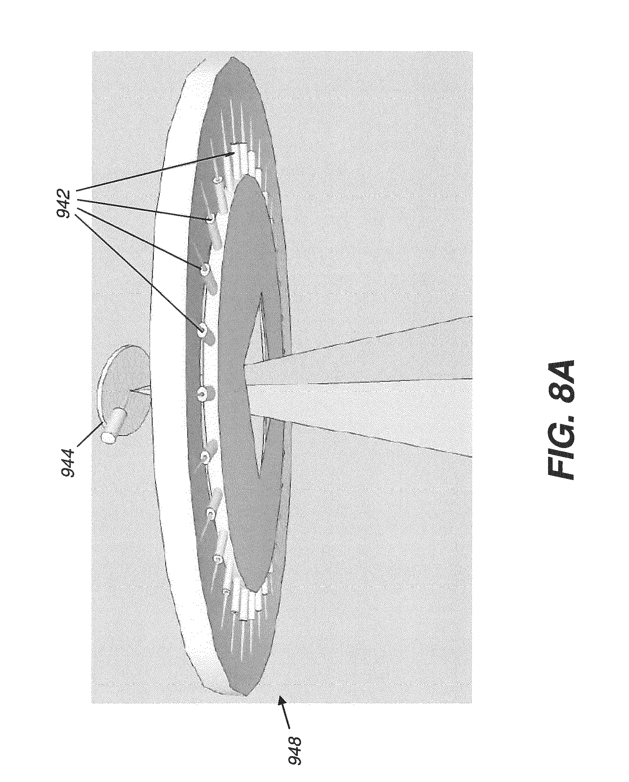

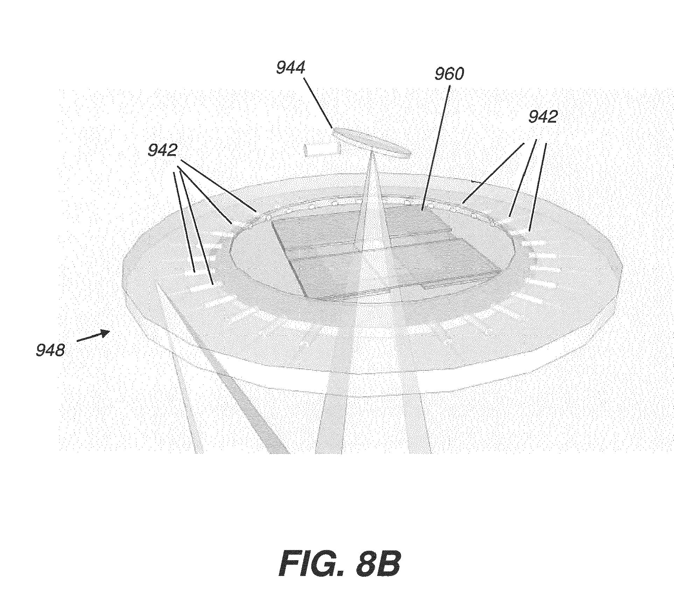

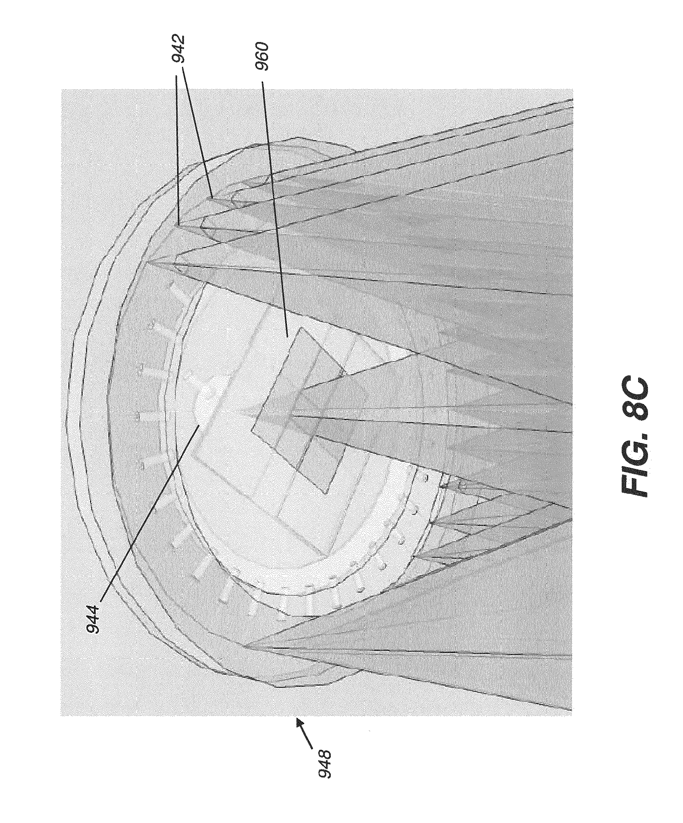

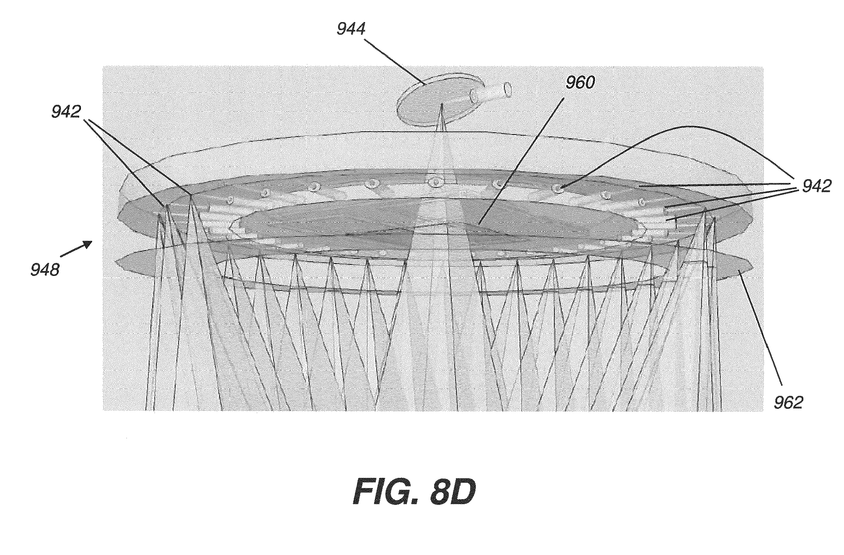

FIGS. 8A, 8B, 8C, and 8D show various embodiments of source array 948 with sources 942 and source 944. A collimator 960 is provided for source 944. Additional collimators 962 provide collimation for sources 942. According to an embodiment of the present disclosure, each individual source 942 has its own collimator 962, as in the embodiment shown in FIG. 8D, for example. A number of subsequent figures intentionally omit showing the collimator 962 in order to show other details more clearly.

### Sequencing of X-ray Sources

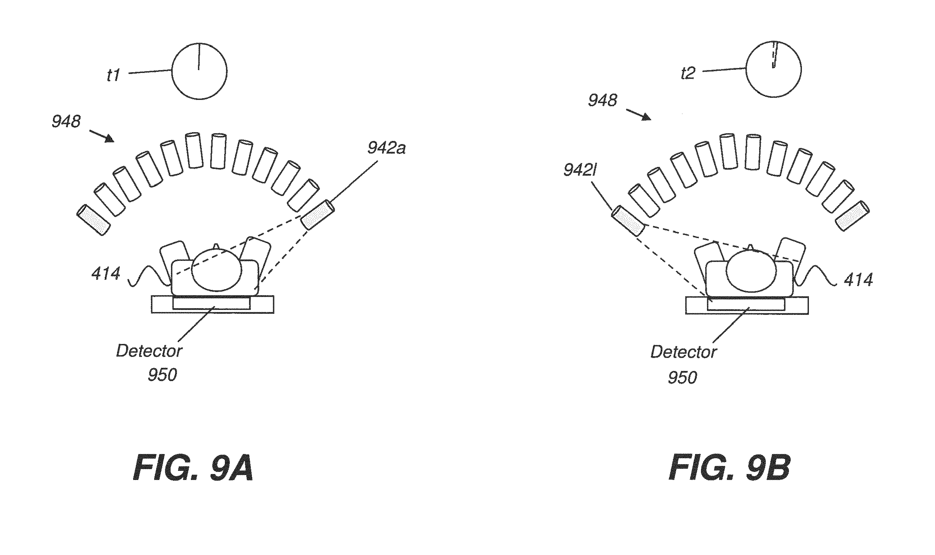

When using source array 948 with sources 942 that are capable of rapid switching, a number of sequencing arrangements can be used for stereoscopic imaging. Referring to FIGS. 9A and 9B, source array 948 is shown in an arcuate arrangement. At a time t1 in FIG. 9A, source 942a is energized and detector 950 acquires image data for a left-eye image. At a different time t2 in FIG. 9B, such as immediately following time t1, source 9421 is energized and detector 950 acquires image data for a right-eye image. The angle between sources 942 used for the left- and right-eye image can be suitably selected to provide a stereoscopic pair. The angular relation can be normal, specified according to the typical separation between eyes for the average sized human adult, for example. Alternately, the angular relation can provide hyperstereoscopic presentation, with abnormally large angular separation between sources, or hypostereoscopic presentation, with abnormally small angular separation between sources.

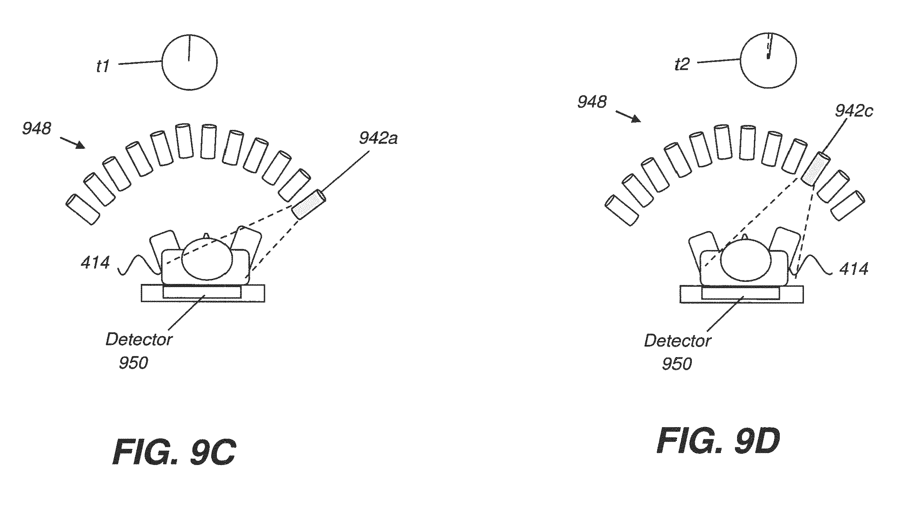

FIGS. 9C and 9D show an alternate selection for a stereo pair, with both sources 942a and 942c, respectively, energized at times t1 and t2 to provide a stereo pair for viewing from the right side of the patient.

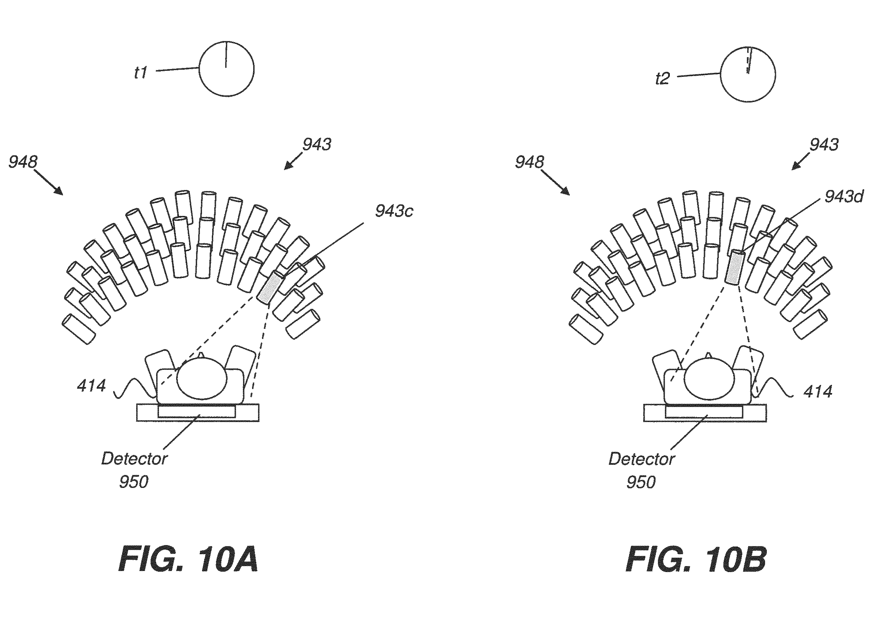

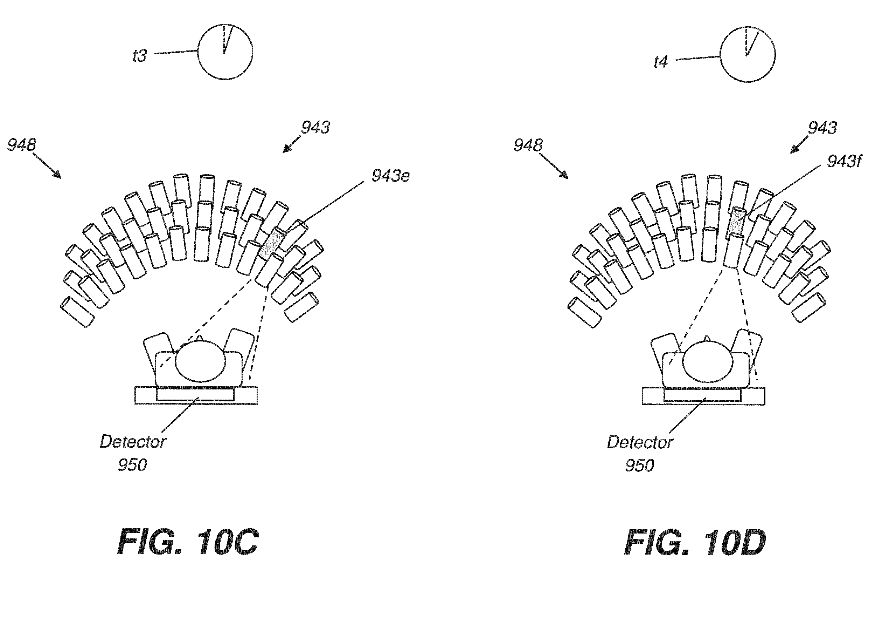

In the imaging arrangements shown in FIGS. 9A, 9B and FIGS. 9C, 9D, images are repeatedly acquired by alternately energizing the same sources. Collimators for individual sources are not shown in FIGS. 9A-9D, but would be provided. Sequencing is also possible changing the pattern of sources. FIGS. 10A, 10B, 10C, and 10D show an exposure sequence in which source array 948 has a large number of sources 943 extending along an arcuate dome above the patient 414. Here, the sequence of exposures progresses along the body of the patient. At time t1, as shown in FIG. 10A, exposure is obtained by energizing a source 943c and acquiring image data. At time t2, as shown in FIG. 10B, exposure is obtained by energizing a source 943d and acquiring image data. The images from sources 943d and 943d form a first stereo pair of images. At a time t3, as shown in FIG. 10C, a source 943e is energized and image data acquired. A second stereo pair of images is formed from the source 943d and 943e exposures. At a time t4, as shown in FIG. 10D, a source 943f is energized and image data acquired. A third stereo pair of images is formed from the source 943e and 943f exposures. This same sequencing pattern can continue, such as along the length of a patient limb or artery, for example. A sequence can be used to track progress of a contrast agent or to examine features that have a particular shape or extension, for example.

For a sequence of images obtained as described with reference to FIGS. 10A - 10D, the stereo pair that is displayed can change with each image that is acquired. Using the sequence of FIGS. 10A-10D for example, the pattern of stereo pairs can proceed as follows:
After acquisition at time t2: a first stereo pair using images from sources 943c and 943d;
After acquisition at time t3: a second stereo pair using images from sources 943e and 943d; and
After acquisition at time t4: a third stereo pair using images from sources 943e and 943f.

This same pattern, in which only one of the left- and right-eye images changes at a time in the displayed stereo pair of images, can repeat to form subsequent stereo pairs for as many sources as are energized in sequence. This type of pattern effectively increases the frame rate for stereoscopic image refresh. Collimators for individual sources are not shown in FIGS. 10A-10D, but would be provided.

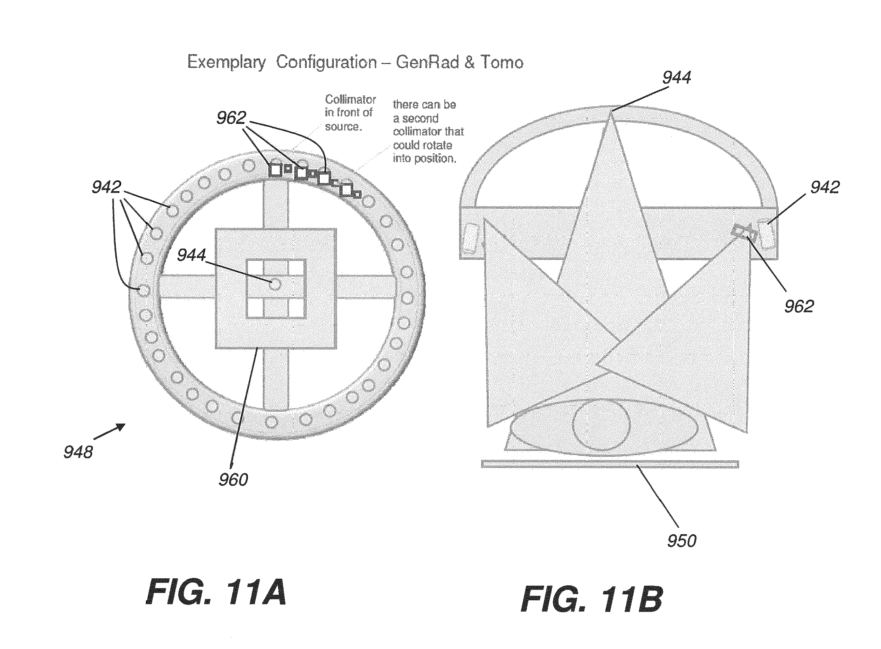

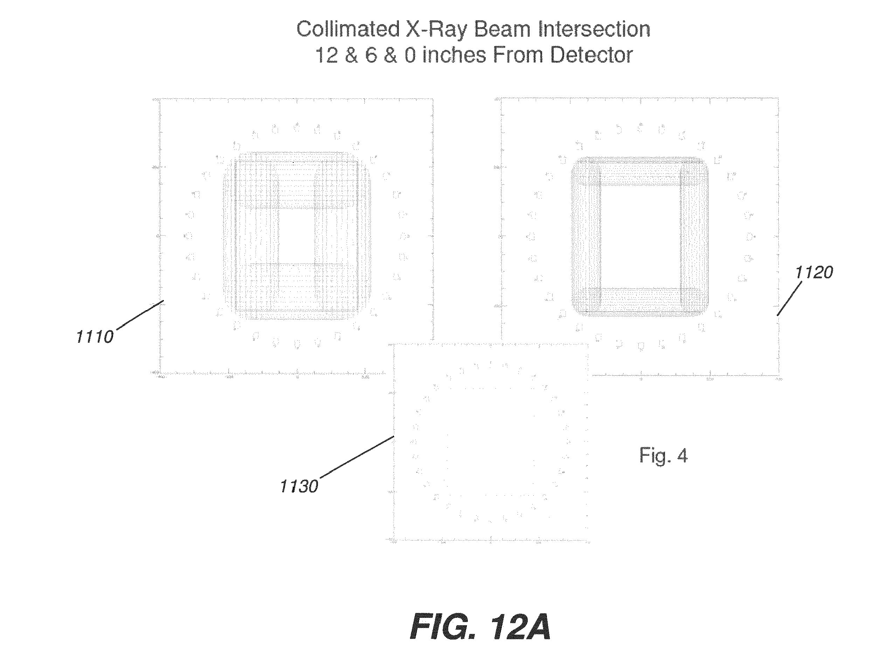

FIG. 11A shows, from a top view, an exemplary configuration of radiation sources 942 for radiography and tomosynthesis imaging similar to those shown in FIGS. 8A-8D. FIG. 11B shows a side view. The schematic view of FIG. 12A shows the intersection of collimated x-ray beams at different distances from the detector 950. At a position 1110, the beam intersection at 12 inches from the detector is shown. At a position 1120, the beam intersection at 6 inches from the detector is shown. At a position 1130, the beam intersection at the detector is shown.

In one embodiment, the arranged or distributed low power source can be an array of carbon-nanotube x-ray sources. In one embodiment, a plurality or all of the electron beams emitted by the carbon nanotube sources arranged in the circle, are directed at a single, shared anode. This anode embodiment can be a disc with a hole in the center. For example, the anode embodiment can have a beveled edge so the electron beam can impinge the anode embodiment at the correct angle for x-ray emission. The anode embodiment (e.g., disk) can rotate so the points where the electron beams hit trace out line segments that can distribute the energy over a larger surface area of the anode to reduce damage (e.g., overheating, melting).

Certain exemplary embodiments shown in the figures also illustrate a central x-ray source 944 with a more traditional collimator 960. This central x-ray source can be used to capture traditional x-ray images. Further, the central x-ray source can also be used as one of the distributed source to capture the multiple projections x-ray images that can be processed to obtain a limited angle tomosynthesis dataset (e.g., by applying reconstruction processing to that data). The central x-ray source can also use an anode that can move to reduce heating.

Although a circular arrangement of distributed low power x-ray sources are shown here, other linear or non-linear arrangements or even prescribed patterns (e.g., shapes, stars, diamonds, regular or irregular combinations, repeating) can be used with corresponding selectable array of collimation windows that can provide combined tomosynthesis and projection x-ray imaging.

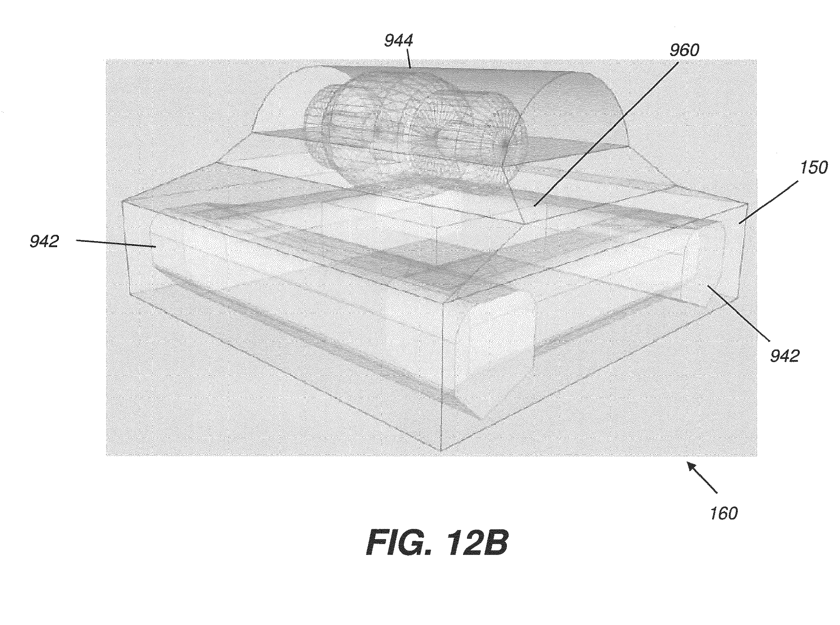

In one exemplary embodiment, a standard radiation x-ray source (or at least one distributed source with standard radiation capabilities) can be enclosed in a single radiation shield with a plurality of distributed sources. For example, a carbon-nanotube (CNT) array tube can be packaged along with a traditional tube in the same housing. In one configuration, a single radiation shield enclosing the combination of sources can provide exterior access to the standard radiation x-ray source without disturbing the additional plurality of low power distributed sources. FIG. 12B shows an embodiment of a radiation shield 150 enclosing a combined tomosynthesis and general radiology (e.g., projection x-ray imaging) x-ray source 160. Further, the embodiment shown in FIG. 12B can incorporate a selectable or re-positionable collimator for the tomosynthesis imaging source and a separate collimator for the general radiology imaging source.

In one embodiment, a single radiographic source can provide both tomosynthesis and general radiology (e.g., projection) x-ray imaging. For certain exemplary embodiments, the single radiographic source comprises at least two collimators. For example, the at least two collimators can include a first collimator for the general radiology source that is fully adjustable (e.g., 3D x-ray beam shaping) and a second collimator for tomosynthesis imaging that can used limited beam shaping capability (e.g., two directions, two distances or two apertures). A tomosynthesis imaging source can be an array of distributed source such as a line or ordered sequence (e.g., linear or non-linear) of low power sources (e.g., CNT x-ray sources). In one embodiment, the second collimator can be a tube around the tomosynthesis imaging source that can arrange (e.g., rotate, slide) at least two apertures into place for beam shaping. In another embodiment, the second collimator can be a corresponding unit that can move in or out (with one or more apertures) relative to the tomosynthesis imaging source for beam shaping. In another embodiment, the second collimator can be a corresponding unit that can removably attach (e.g., snap in, twist in, hingeable, or twist fastener) to the single source at a plurality of positions or locations for tomosynthesis imaging source beam shaping. In one embodiment, the first and second collimators can be discrete adjustable units or a combined unit. In one embodiment, both the first and second collimators can be within a single radiation shield. Alternatively, one of the first and second collimators can be within the single radiation shield and the other collimator can be outside.

Two different type of x-ray sources (i) general radiation source and (ii) distributed array of certain number of sources (e.g., lower power) can be included in a single x-ray source for a radiographic imaging system according to embodiments of the application.

One exemplary embodiment for the distributed array of sources can be a configuration that can include 3-20 distributed sources in a unit (e.g., unit array of distributed sources) at sides (e.g., each of 3-8 sides around a central area) to make an arrangement, which configuration can be separated and individually attached by unit array (or fastened together in a single entity) to a mechanical housing (e.g., tube head) of the imaging system. For certain exemplary embodiments, the unit arrays are not co-planar and can implement a different source-to-image distance (SID) for an imaging event or examination. For example, the unit arrays can be selectively co-planar, for example, two sides at different depths, three of four sides at different planes. Further, the (vertical, horizontal) distance between the unit arrays can be the same or different (e.g., increasing). Alternatively, adjacent or opposite pairs of unit arrays can have equal SIDs or be co-planar. Such a variation in arrangement can allow for a fixed x-ray source arrangement to implement a greater range of subject distances.

By implementing a distributed source in several smaller pieces, certain exemplary embodiments can include independent movement of the plurality of unit arrays of distributed sources. For example, one exemplary embodiment can include a configuration that can make the unit arrays (e.g., four arms) independently adjustable or able to move separately. Thus, individual unit arrays or opposite unit arrays can be moved outward to provide a wider angular coverage to improve in plane or out of plane resolutions. In one embodiment, such outward movement of at least one unit array can be accompanied by additional adjustment of the unit array to maintain or achieve a desired arrangement or overlapping of x-ray beams from the unit arrays at a DR detector. For example, individual unit arrays can rotate independently (e.g., two opposing edges can rotate outward for an increased SID) to adjust for different SIDs (e.g., increased or decreased) to bring into overlapping on the detector (e.g., focus). In other words, such movement can be included with collimation adjustments by rotating a collimation aperture or switching between a plurality of collimation apertures. However, such rotation can compensate for change in an x-ray beam in one dimension (e.g., X-direction or the Y-direction) as the SID changes. In one embodiment, an additional collimation can be used at a distance closer (e.g., 6 inches-2 feet) to the detector to provide an outer limitation to the collimated beams of the distributed array of sources.

Certain exemplary embodiments can include independent movement of the plurality of unit arrays of distributed sources to implement different examinations or SIDs. For example, a chest x-ray examination can use a longer SID than a head x-ray examination and accordingly, movement (e.g., spatial positioning and/or rotation) of the unit arrays can allows multiple distances or SIDs to be implemented with a single aperture (e.g., fixed collimation, pinhole) for each distributed source.

Certain exemplary embodiments can include independent movement of the plurality of unit arrays of distributed sources to implement different formations on a mobile x-ray imaging cart or a portable x-ray imaging system. Thus, independent movement of the plurality of unit arrays can use an extended formation of unit arrays that can have a significant length (e.g., 3-8 feet) in an imaging configuration for a mobile x-ray cart that can fold or disassemble into a reduced size or 3D footprint to allow the mobile x-ray cart to fit into small areas and though doorways.

In one embodiment, the unit arrays can be attached, adjusted and/or removed without tools. In one embodiment, the unit arrays can be attached and/or rotated between two positions where a first position is outside an area traversed by a central x-ray beam (e.g., general radiology beam) and a second position to cross or cover the area traversed by the central x-ray beam. The second position in such a configuration can reduce an angular disbursement of beams from the distributed array of sources.

In one embodiment, a plurality of unit arrays (e.g., 6-8 unit arrays) can be implemented to move between a small retracted configuration and unfold multiple times to form a prescribed linear or non-linear configuration (e.g., multiple straight lines of sources or unit arrays), which can extend in multiple directions from/around a central beam.

In one embodiment, a plurality of unit arrays (e.g., 6-8 unit arrays) can be implemented as individual straight lines sources, but configured to approximate a circle.

Exemplary system and/or method embodiments according to the application can be used for in-room radiographic imaging systems and/or portable tomosynthesis. Portable tomosynthesis imaging may be able to provide the sought information at the bedside without subjecting the patient to the risks of transport to radiology. For example, tomosynthesis imaging can supply the required information to diagnose patient conditions that are non-differentiable with standard projection x-ray imaging such as chest x-rays.

### Digital Radiography (DR) Detector

The x-ray detector can be a digital x-ray detector with signal to noise ratio performance at low exposure to allow readout of the exposure sequences. According to an embodiment of the present disclosure, a DR detector for stereoscopic imaging has a very high frame rate. For example, the x-ray detector can have a frame rate of about 30-60 frames per second. The DR detector should have excellent signal to noise ratio performance at low exposure to allow rapid readout of the rapid exposure sequences. According to an embodiment of the present disclosure, the digital detector employs sensors of complementary metal-oxide semiconductor (CMOS) technology.

The DR detector is independent from the x-ray source array, so that it can be positioned separately. This arrangement allows stereo images to be obtained from any of a number of different view angles, and allows the angular relationship of images to be dictated by the source array arrangement and source-to-image distance (SID), rather than being fixed, such as is required with C-arm arrangements.

### Image processor

The image processing logic must be capable of rapid spatial frequency processing. Lag time between image acquisition, processing, and data transmission must be reduced to low levels, so that response and refresh time of the DR detector and associated components is as low as possible.

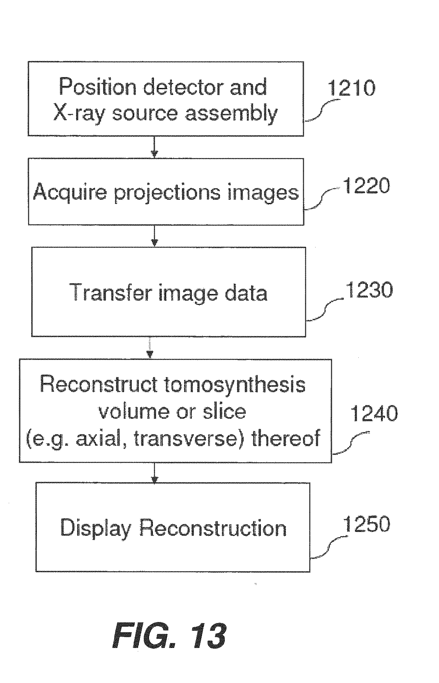

The flow chart of FIG. 13 shows an exemplary method for acquiring projection images and reconstruction of three-dimensional tomosynthesis images, according to an embodiment of the present disclosure. The method described can use embodiments of mobile radiography apparatus shown in FIG. 5, for example. Methods for stereoscopic image capture, processing, and presentation given in the present disclosure can be applied to other mobile or stationary imaging apparatus, without limitation to a particular type of system.

As shown in FIG. 13, in a positioning step 1210, the detector and x-ray source array can be positioned. For example, the x-ray source can be moved to its initial position and the detector can be positioned such that the patient P is interposed between the detector and x-ray source.

For an embodiment of exemplary mobile radiographic/tomosynthesis unit 900 of FIG. 5, the initial x-ray source array position can be set by the location of transport frame 920 and the support column 930 to which the source array is coupled. The height, extent and rotation positioning of first section 930a and second section 930b of support column 930, or positioning elements that are themselves connected to support column 930, can be used to position the x-ray source array 940 to the initial desired location with respect to the patient.

Following positioning step 1210 in FIG. 13, an image acquisition step 1220 acquires a series of projection image at different x-ray source positions. Each of the projection images can be acquired while corresponding individual x-ray sources are triggered. In one embodiment, the first radiographic x-ray source can operate as a central one of the distributed sources. In a transfer step 1230, the acquired projection image data can be received (e.g., transferred back from the detector to the system) by control and processing components of the system controller. The projection images can be displayed on display 910 and/or undergo a quality check (e.g., automated or by the operator) before being further processed. The projection image data may also be processed in transfer step 1230 to permit raw, partially-processed or fully-processed images or tomosynthesis slices to be stored (e.g., to support temporality at the detector) and/or sent to remote locations.

Continuing with FIG. 13, tomosynthesis image reconstruction can be performed (e.g., real-time) using the acquired corrected projection image data in a reconstruction step 1240. Image reconstruction can use processes similar to those used for conventional tomosynthesis imaging. For example, as will be appreciated by those skilled in the art, back projection, filtered back projection or other known reconstruction techniques may be used. In one exemplary embodiment, a particular position of the source with respect to the detector can be determined by knowledge of the position of the x-ray source array and the detector based upon the values set by an operator, or automatically determined according to image capture timing or by using a grid alignment system to adjust the values or by a tethered connection to source and detector positioning circuitry, for example. The reconstructed volume is provided on display 910 in a display step 1250.

The reconstructed volume can alternately undergo a quality check before display. In one embodiment, the reconstruction volume can be stored after the quality check (e.g., before display). Further, the display can be used to view underlying projection images or projection images generated by the system, or to view the tomosynthesis reconstructions themselves. Further, underlying data and/or reconstructed tomosynthesis images can be transmitted to a remote system for additional analysis or display.

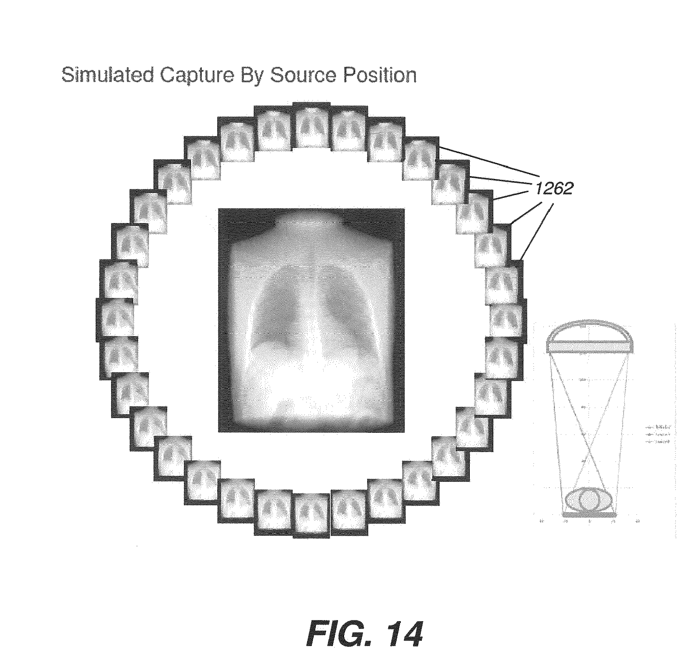

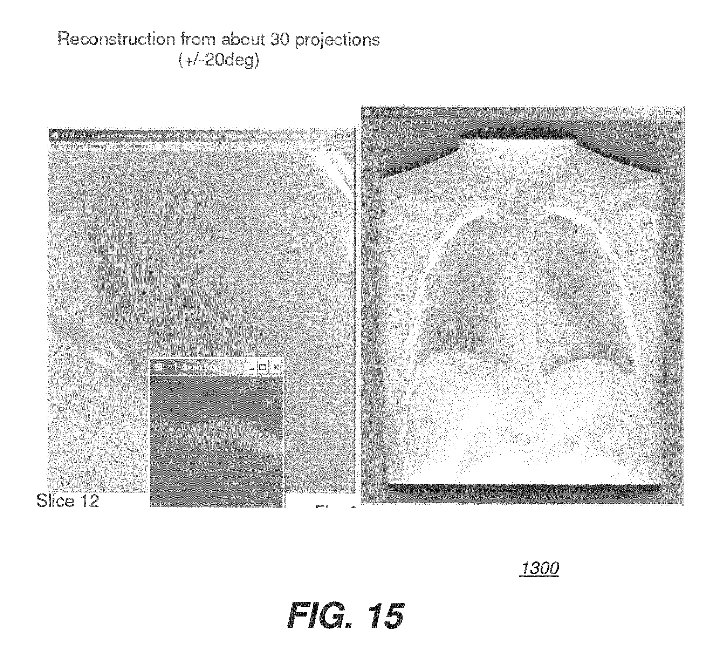

FIG. 14 shows simulations of exemplary projection images 1262 obtained using x-rays from each source position. It should be noted that FIG. 14 shows a full 360 degree orbit for obtaining projection images 1262. In practice, images are acquired over a much smaller range for tomosynthesis imaging. FIG. 15 shows a tomosynthesis reconstruction image 1300 according to an embodiment of the present disclosure.

The tomosynthesis image 1300 displays to the practitioner as a 2-D slice extracted from the volume data. Stereo imaging requires 2-D slices taken from slightly different angles.

### Stereoscopic display apparatus

Stereo display systems, also termed stereoscopic display or viewing apparatus, are known, using various arrangements for providing separate and complementary left-eye and right-eye images. A stereoscopic display apparatus would enable interventional radiologists to visualize depth while performing procedures, such as procedures that involve complex positioning and movement.

For stereoscopic imaging, the left-eye image and right-eye image can be separated, so that they are perceptibly distinct to right and left eyes of the viewer, on a display system using approaches that include:
(i) Alternating timing. In a time-phased approach, the right- and left-eye images are rapidly alternated on the display. Viewing apparatus worn by the viewer, such as stereoscopic viewing goggles or glasses, for example, are then coordinated with the display to toggle left- and right-eye visibility with corresponding timing. Alternating shutters can be used for this purpose.
(ii) Orthogonal polarization. Left- and right-eye images can be distinguished from each other using polarized light. With this type of approach, stereoscopic goggles or glasses have polarizers that correspondingly block and allow light of orthogonal polarization states. Image content for the left eye is formed at a first display from light of a first polarization transmission axis; image content for the right eye is formed at a second display from light of a second polarization transmission axis that is orthogonal to the first polarization transmission axis. Polarization can be linear, elliptical, or circular, with modulated light for the left eye orthogonally polarized with respect to modulated light for the right eye. The displays can be separate displays, one for the left-eye image and one for the right-eye image; alternately, a single display with interleaved polarized light emission by pixel, or by group of adjacent pixels, can be used.
(iii) Spectral separation. Largely used with color imaging projectors, spectral separators separate left- and right eye images by transmitting different spectral bands or spectral ranges for the component colors that are directed to each eye. Where red, green, and blue filters are used, for example, red spectral content for the left eye may be within the 620-630 nm band. Red spectral content for the right eye would be within the 640-650 nm band. Complementary filters would be used for transmitting and blocking the light content appropriately for each eye. Spectral separation can be provided from different display or projection devices or from a single device operating in an interleaved mode.

In addition, various types of lenticular displays can alternately be used to provide stereoscopic image pairs, such as the displays described in US Patent Publication No. 2012/0250151 (Lee) entitled "Lenticular unit for two-dimensional/three-dimensional auto-stereoscopic display".

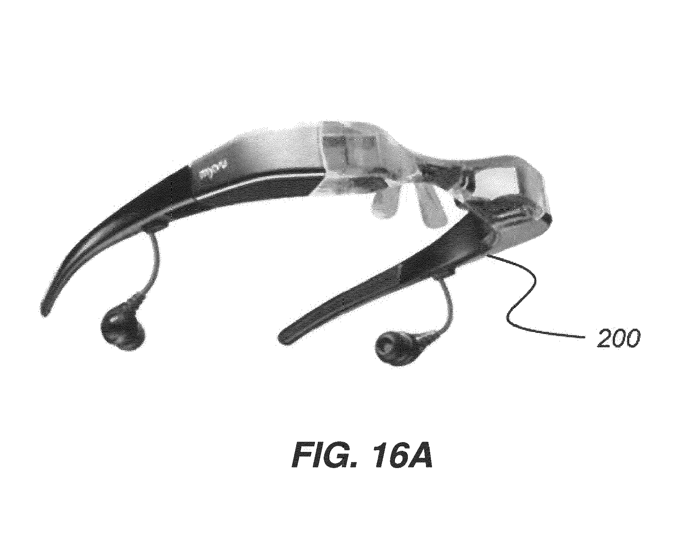

FIG. 16A shows a perspective view of stereoscopic viewing glasses that provide a type of wearable stereoscopic viewing apparatus 200. For stereoscopic viewing, eyeglasses, goggles, and similar wearable devices can use shuttering, polarization, or spectral separation for separate left- and right-eye image presentation.

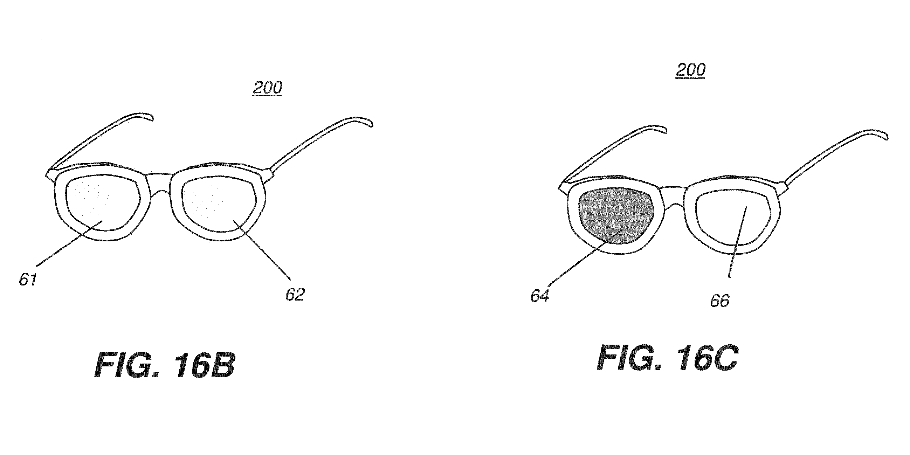

FIG. 16B shows wearable viewing apparatus 200 having orthogonal polarizers 61 and 62.

FIG. 16C shows wearable viewing apparatus 200 with spectral filters 64 and 66.

Other methods for stereoscopic image display may use an arrangement of curved reflectors for presenting a virtual image to the practitioner or other viewer. With systems of this type, wearable viewing apparatus 200 would not be needed; it would be required, however, to have the viewer's pupils within a narrow spatial region in order to provide the virtual image. Virtual image presentation is taught, for example, in U.S. Patent 6,416,181 (Kessler) entitled "Monocentric autostereoscopic optical apparatus and method".

Particularly suitable for an embodiment in which timing is used for stereoscopic image separation (i, above), the system creates stereo pairs by generating rapid sequential exposures from each of the two x-ray sources that are spatially separated. Each image of a pair is read out from the x-ray detector, pixel corrections and image processing applied, then the stereo pairs are displayed on the stereo viewer. This process is continuously repeated in rapid succession to form a motion sequence of stereo pairs. The system can be coupled with a power contrast injector in the same manner as-is done today with conventional IR systems. The stereo pair can be displayed using various sequences, such as: 1-2, 3-4, 5-6, etc., wherein each stereo pair includes a newly displayed left-eye image and a newly displayed right-eye image. Alternately, a sequence of stereo pairs in which only one of the complementary images is updated at a time can be displayed, using a sequence such as 1-2, 2-3, 3-4, etc., as described previously with reference to FIGS. 10A-10D. Imaging enhancement processing might be applied such as sharpness adjustment, and temporal smoothing, noise reduction, flicker reduction, tonal adjustment, and the like. With this timing sequence, the same image content for one eye can be displayed a number of times until it is refreshed, alternated with the corresponding image content for the other eye. This same type of rapid image sequencing can also be used where spectral separation, lenticular display, or polarization is used for providing complementary stereoscopic images.

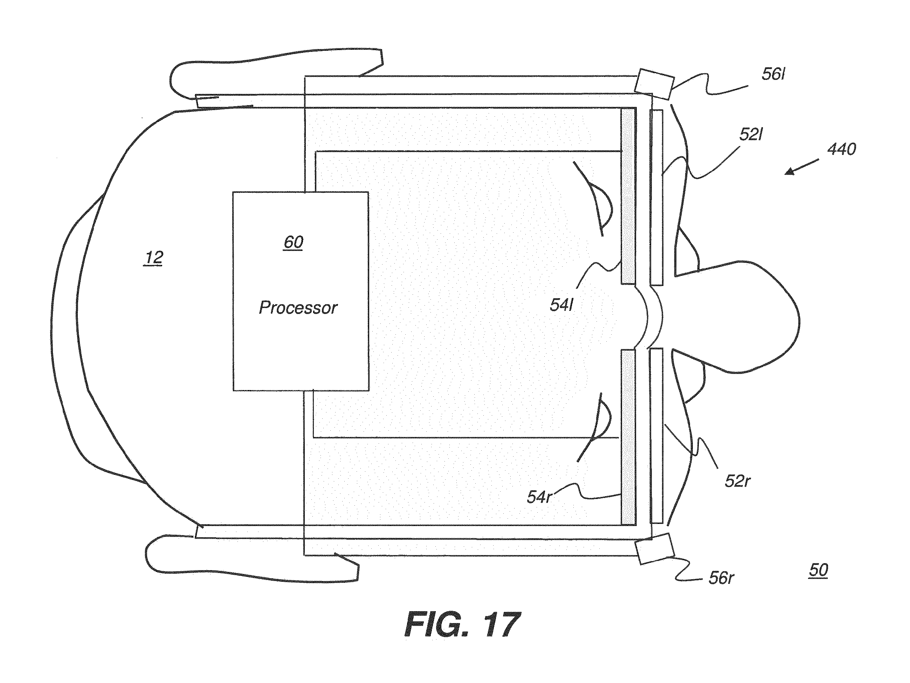

Another type of wearable stereoscopic viewing apparatus 440 can be a head mounted device, as shown in the top view of FIG. 17. Head mounted devices (HMDs) 50 are known to those skilled in the visualization art and operate by displaying a computer-generated image to the left and right eyes of an observer 12, such as the practitioner or other viewer who wears the device. HMD devices 50 can act not only as displays, but also as interactive devices for improved visualization and viewer interaction with an imaging system. The displayed image on HMD 50 can be stereoscopic and can be provided so that the image display is at least partially transparent, so that the viewer can see the displayed image and the real-world image at the same time through the HMD lenses. The displayed, computer-generated image can serve as a type of reference during examination and treatment.

Viewing of both real-world and computer-generated images can be executed in any of a number of ways. According to an embodiment of the present invention, display elements 541 and 54r have pixels spaced apart so that the computer-generated image only obstructs a portion of the real-world view through lenses 521 and 52r and both computer-generated and real-world views can be visible at the same time. According to an alternate embodiment of the present invention, the computer-generated view is opaque, and the display that appears on display elements 541 and 54r is rapidly alternated with a clear display through lenses 521 and 52r, such as 20 times per second or more, so that the appearance of simultaneous viewing is provided to the HMD viewer.

Still referring to FIG. 17, lenses 521 and 52r can be standard prescription lenses. Display elements 541 and 54r of HMD 50 can be devices that incorporate a spatial light modulator, such as a digital micro-mirror array or similar device, or can be emissive devices, such as organic light-emitting diode (OLED) arrays, for example.

Gaze tracking and other methods can be used to detect head or eye movement for the person wearing the HMD and to report changes to a processor 60 so that the displayed stereoscopic images can be adjusted or other appropriate functions performed. Processor 60 can perform some or all of the functions assigned to the image processor, including generation of the stereo images displayed on HMD 50. Alternatively, processor 60 can be in signal communication with another processor, such as processor 430 in FIG. 4, for participating in image acquisition, processing, and display functions.

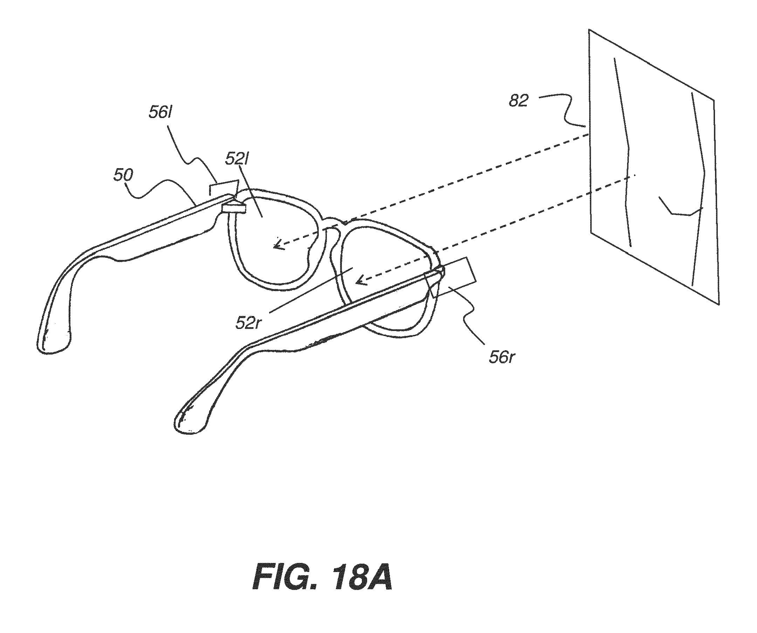

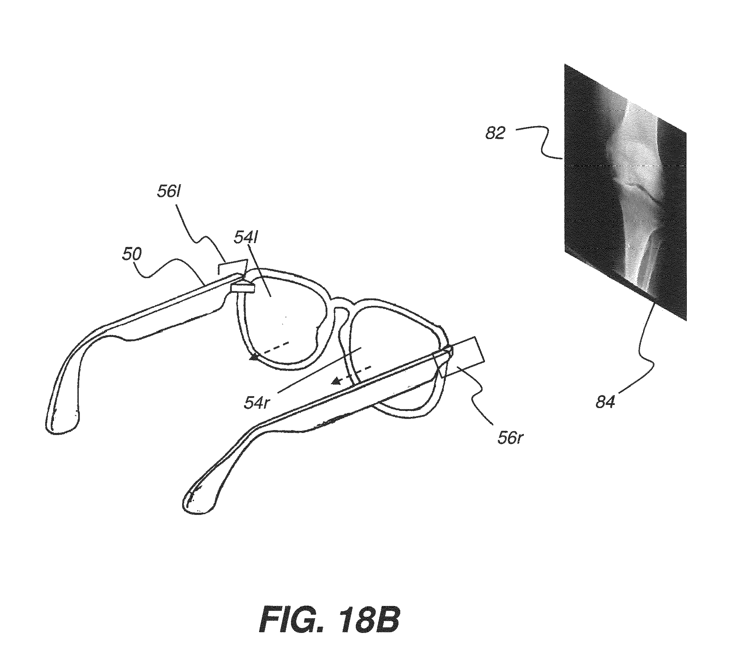

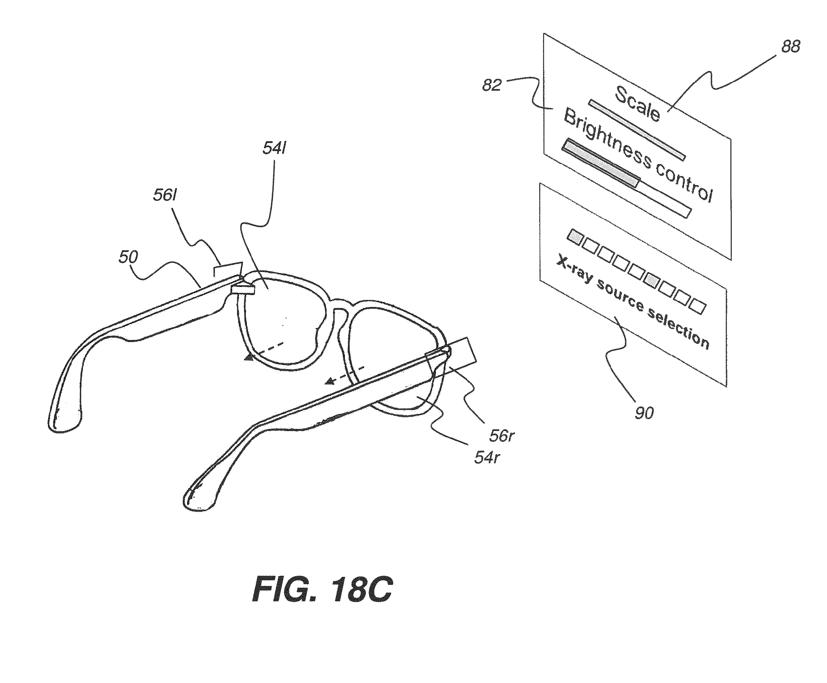

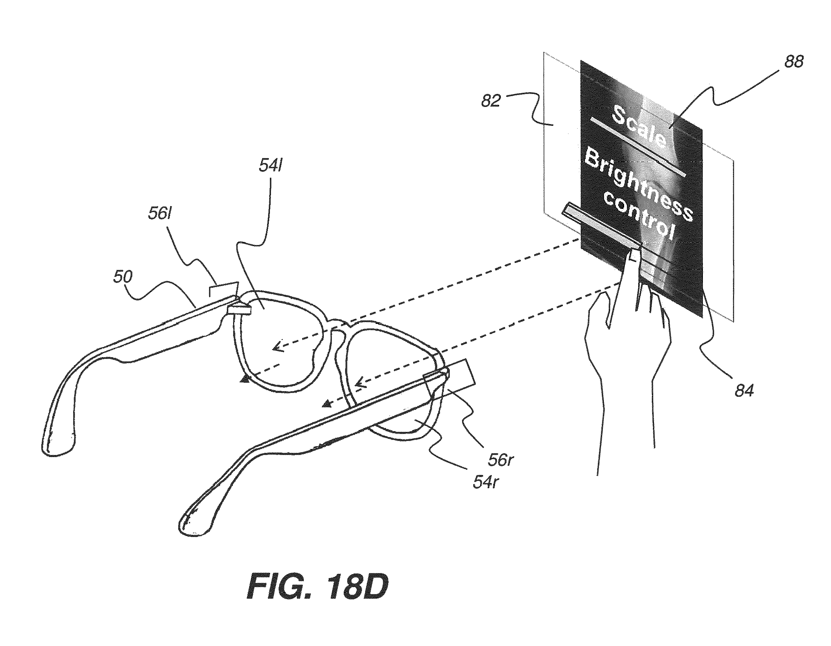

The combination of lenses 521, 52r, display elements 541, 54r, and cameras 561, 56r enables HMD 50 to perform a number of visualization functions simultaneously. FIGS. 18A, 18B, 18C, and 18D show some of the separate and combined visualization functions that are provided. FIG. 18A represents the actual view of patient anatomy in a field of view 82 as seen through lenses 521 and 52r. The doctor can switch between transparency of lenses 521 and 52r for viewing the patient or viewing an external display and stereoscopic display using HMD 50. FIG. 18B shows a stereoscopic radiographic image 84 that corresponds to the patient anatomy of interest for the patient who is in field of view 82. Image 84 is displayed on display elements 541 and 54r to present a stereoscopic image. FIG. 18C shows a computer-generated image of controls 88 for image display as provided from processor 60 (FIG. 17) and operational controls 90 for entering operational parameters, such as specifying the radiation sources within the array for left- and right-eye imaging. In the example shown, the computer-generated image shows user interface controls 88 for image scale and brightness. These operator interface controls 88 are displayed by left and right display elements 541 and 54r. In addition, or in alternating fashion, stereoscopic image content can also display in stereoscopic form from left and right display elements 541 and 54r, respectively, so that both radiographic and user interface images shown in FIGS. 18B and 18C are displayed to the viewer, who also has the ability to see the patient during imaging operation as was described with respect to FIG. 8A. FIG. 18D shows the combined view with the stereoscopic computer-generated image of controls 88 superimposed on stereoscopic image 84 in field of view 82.

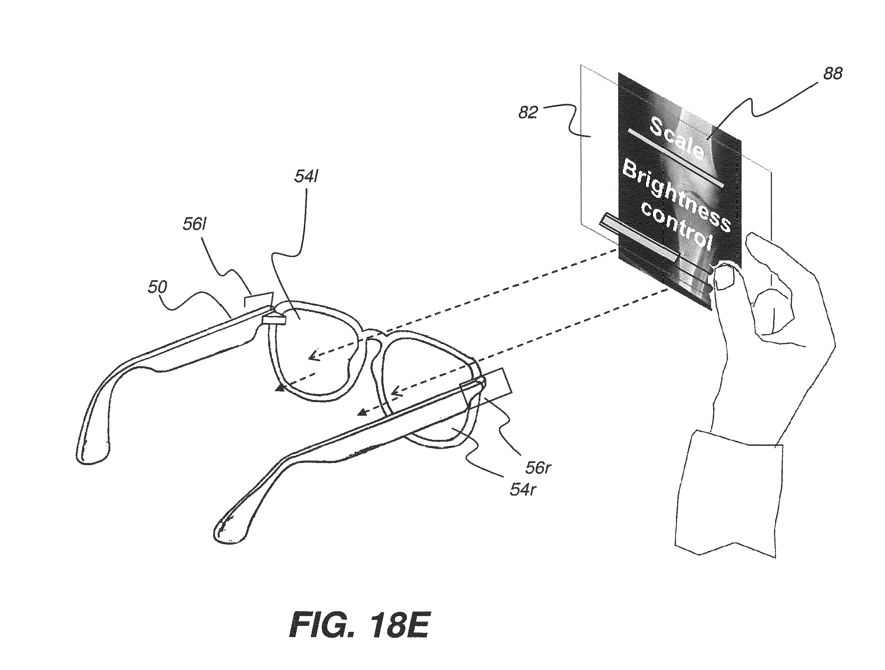

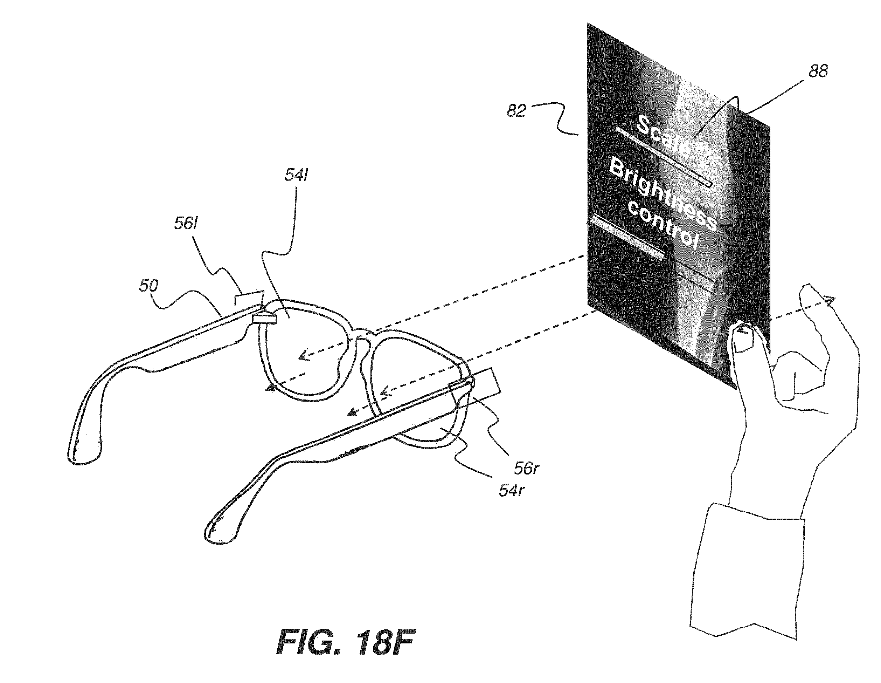

HMD 50 also allows the capability to enter operator gestural commands as part of the user interface. FIGS. 18E and 18F show a gestural instruction that changes the scale of a displayed reference image, allowing variable zoom and pan functions or paging through multiple windows that allow command or parameter entry, for example. Gestural commands can also be used to control aspects of mobile radiography apparatus 100 operation, including selection of x-ray sources, as described subsequently.

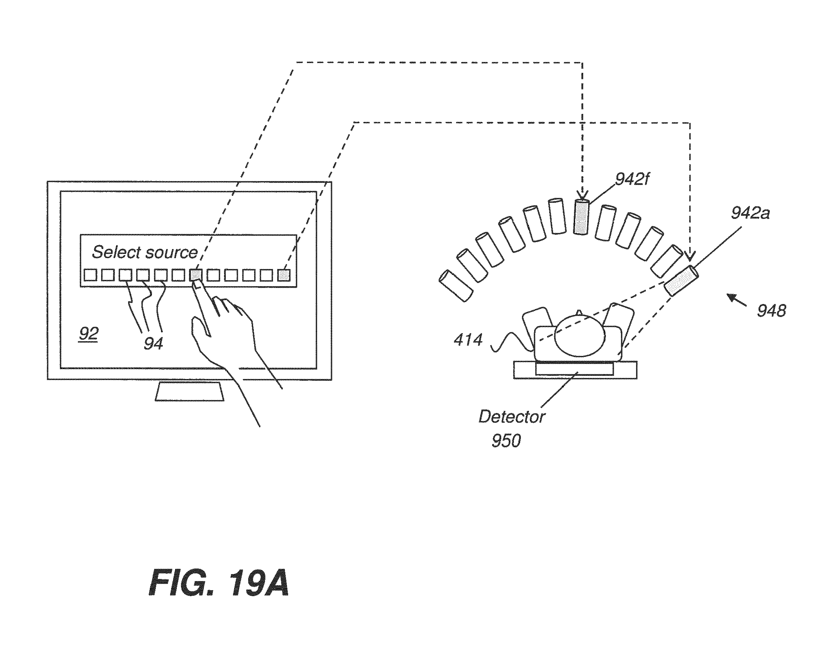

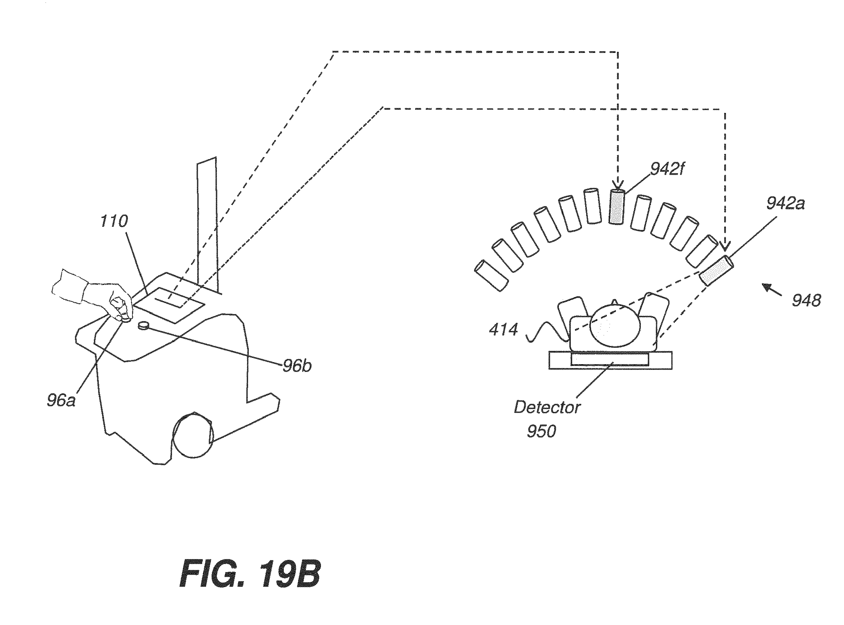

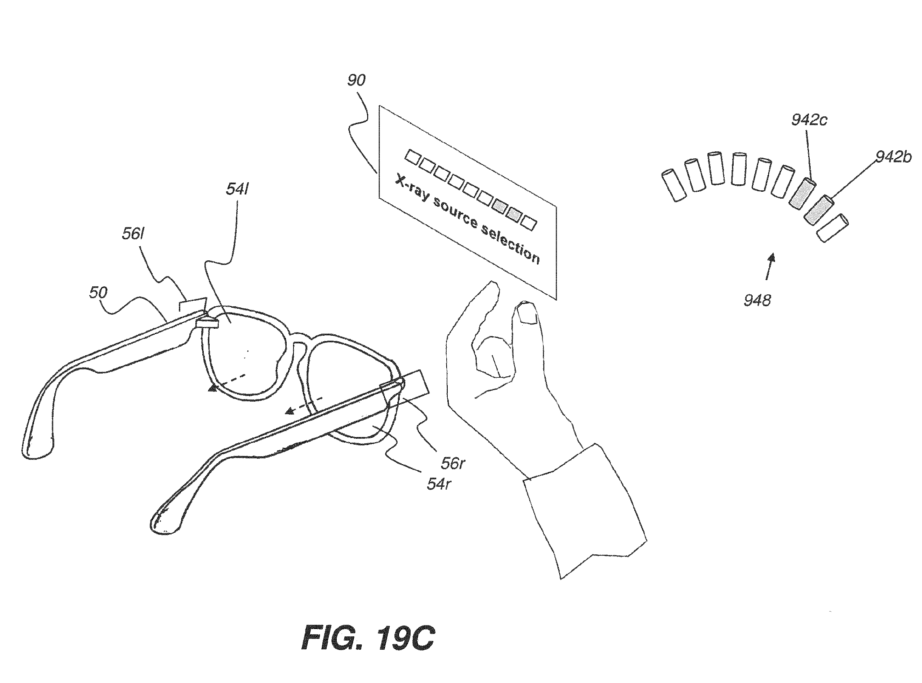

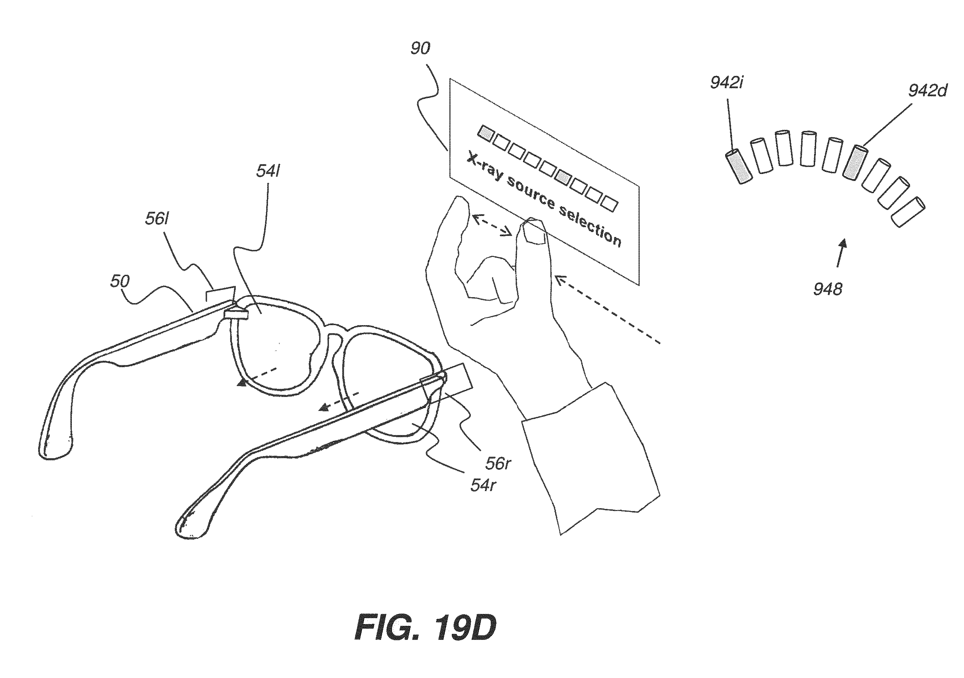

The operator of the imaging apparatus has the capability to specify which x-ray sources in x-ray source array 948 are to be energized for providing the left-eye and right-eye stereoscopic radiographic images. One or more controls are configured to enable operator selection of two or more individual x-ray sources from the x-ray source array. This operator-selectable feature for specifying x-ray sources enables the operator to change the view angle and right-left separation distance for stereoscopic imaging. FIGS. 19A, 19B, 19C, and 19D show various control mechanisms for making this adjustment.

In FIG. 19A, an operator interface screen 92, such as on display 110 (FIG. 1) provides symbols 94 corresponding to each x-ray source in array 948. In the example shown, interface screen 92 is a touchscreen that allows the operator to select an x-ray source from array 948 to be enabled as the source for a left-eye image, shown as source 942f. Another selection on this touch-sensitive device allows operator selection of the source enabled for the right-eye image, shown as source 942a.

FIG. 19B shows an alternate embodiment for source selection to get the desired view angle and separation using a control knob 96a for one of the sources and a second control knob 96b for the other source (or, equivalently, using control knob 96b for setting the relative distance between the two sources). According to an alternate embodiment of the present disclosure, x-ray source selection for specifying the view angle and separation distance can be performed using a joystick, mouse, touchpad as used on a laptop computer, or other touch-sensing device to provide a suitable signal. Enablement by audible command can also be used. Display 110 shows the settings for x-ray source enablement according to the signal or signals received from the specifying device. According to an alternate embodiment of the present disclosure, light-emitting diode or other indicators disposed on or near the selected x-ray sources illuminate to indicate which sources have been enabled by the operator.

FIGS. 19C and 19D show the use of HMD 50 for x-ray source selection using gestural commands or instructions from the viewer. In the example shown, the relative position of the viewer's hand and distance between fingers is used to select the relative location for centering the two enabled x-ray sources that are selected and for specifying the distance between them. In FIG. 19C, sources 942b and 942c are selected to be enabled. In FIG. 19D, sources 942d and 942i are selected for enablement.

Particular gestures can be assigned to different functions and customized, so that a particular practitioner uses gestural commands to control aspects of imaging system operation or displayed image content. Operator movement can be observed in a number of ways and postural, positional, and gestural movements can be interpreted for providing instructions. Thus, for example, movement of the observer to another position relative to the patient can be interpreted by HMD 50 as an instruction to modify the presentation of the displayed image, such as changing the perspective view angle. Similarly, gaze-tracking can be used to detect operator eye movement and use this detection to condition the behavior of the imaging apparatus, such as shifting the view angle accordingly. Methods for sensing and interpreting operator gestures and operator movement for instruction entry are known to those skilled in the imaging arts.

By projecting operator controls 88 in field of view 82 and monitoring hand gestures or other visible signals from the observer within the field of view, acquired using cameras 561 and 56r, head-mounted device 50 enables processor 60 to obtain viewer instructions and allows the practitioner to control the display of the stereoscopic images with gestural commands and instructions. Gaze tracking can also be used to adjust image display parameters according to viewer attention.

HMD 50 is in signal communication with the processor that obtains image data and generates stereoscopic image content. This signal communication can be wired or wireless.

One or more digital radiographic imaging detectors can be used in the stereoscopic fluoroscopy apparatus. An arrangement with multiple detectors can be used to provide stereoscopic images, wherein the two detectors are alternately exposed and accessed for digital data.

Consistent with one embodiment, the present invention utilizes a computer program with stored instructions that perform on image data that is accessed from an electronic memory. As can be appreciated by those skilled in the image processing arts, a computer program of an embodiment of the present invention can be utilized by a suitable, general-purpose computer system, such as a personal computer or workstation that acts as an image processor. However, many other types of computer systems can be used to execute the computer program of the present invention, including an arrangement of networked processors, for example. The computer program for performing the method of the present invention may be stored in a computer readable storage medium. This medium may comprise, for example; magnetic storage media such as a magnetic disk such as a hard drive or removable device or magnetic tape; optical storage media such as an optical disc, optical tape, or machine readable optical encoding; solid state electronic storage devices such as random access memory (RAM), or read only memory (ROM); or any other physical device or medium employed to store a computer program. The computer program for performing the method of the present invention may also be stored on computer readable storage medium that is connected to the image processor by way of the internet or other network or communication medium. Those skilled in the art will further readily recognize that the equivalent of such a computer program product may also be constructed in hardware.

It is noted that the term "memory", equivalent to "computer-accessible memory" in the context of the present disclosure, can refer to any type of temporary or more enduring data storage workspace used for storing and operating upon image data and accessible to a computer system, including a database. The memory could be non-volatile, using, for example, a long-term storage medium such as magnetic or optical storage. Alternately, the memory could be of a more volatile nature, using an electronic circuit, such as random-access memory (RAM) that is used as a temporary buffer or workspace by a microprocessor or other control logic processor device. Display data, for example, is typically stored in a temporary storage buffer that is directly associated with a display device and is periodically refreshed as needed in order to provide displayed data. This temporary storage buffer can also be considered to be a memory, as the term is used in the present disclosure. Memory is also used as the data workspace for executing and storing intermediate and final results of calculations and other processing. Computer-accessible memory can be volatile, non-volatile, or a hybrid combination of volatile and non-volatile types.

It is understood that the computer program product of the present invention may make use of various image manipulation algorithms and processes that are well known. It will be further understood that the computer program product embodiment of the present invention may embody algorithms and processes not specifically shown or described herein that are useful for implementation. Such algorithms and processes may include conventional utilities that are within the ordinary skill of the image processing arts. Additional aspects of such algorithms and systems, and hardware and/or software for producing and otherwise processing the images or co-operating with the computer program product of the present invention, are not specifically shown or described herein and may be selected from such algorithms, systems, hardware, components and elements known in the art.

The invention has been described in detail, and may have been described with particular reference to a suitable or presently preferred embodiment, but it will be understood that variations and modifications can be effected within the spirit and scope of the invention. The presently disclosed embodiments are therefore considered in all respects to be illustrative and not restrictive. The scope of the invention is indicated by the appended claims, and all changes that come within the meaning and range of equivalents thereof are intended to be embraced therein.

## Claims

1. A portable imaging apparatus for obtaining stereoscopic radiography images, comprising:
a mobile transport frame having a support structure;
an x-ray source array coupled to the support structure and having at least three x-ray sources, each x-ray source being separately energizable to emit radiation toward a subject;
at least one x-ray detector adapted to acquire digital image data according to radiation received from the at least three x-ray sources of the x-ray source array;
an image processor in signal communication with the at least one x-ray detector and adapted to generate, from the acquired digital image data, a right-eye digital image of the subject and a left-eye digital image of the subject; and
a display apparatus in signal communication with the image processor and adapted to display the right-eye and left-eye digital images of the subject to a viewer.

2. The imaging apparatus of claim 1 wherein the at least three x-ray sources are carbon nanotube x-ray sources.

3. The imaging apparatus of claim 1 wherein the display apparatus comprises stereoscopic viewing glasses.

4. The imaging apparatus of claim 3 wherein the stereoscopic viewing glasses are shutter glasses and wherein the right-eye digital image and the left-eye digital image display over alternating time intervals.

5. The imaging apparatus of claim 3 wherein the stereoscopic viewing glasses have spectral filters and wherein the right-eye digital image and the left-eye digital image differ according to color spectra.

6. The imaging apparatus of claim 1 wherein the display apparatus comprises a head-mounted device wirelessly coupled to the image processor.

7. The imaging apparatus of claim 1 wherein the x-ray source array has (1) a geometrically arcuate distribution of sources or (2) a substantially linear distribution of sources.

8. The imaging apparatus of claim 1 wherein the right-eye digital image has a first polarization state and the left-eye digital image has a second polarization state orthogonal to the first polarization state.

9. A portable imaging apparatus for stereo fluoroscopy of a subject, comprising:
an x-ray source array having three or more x-ray sources, each x-ray source having a fixed position within the array and having a corresponding collimator and independently energizable to emit radiation toward the subject, wherein the array extends from a mobile transport frame and wherein at least two of the three or more x-ray sources are operator selectable;
at least one x-ray detector adapted to acquire digital image data for multiple images from the subject according to radiation received from the x-ray sources;
an image processor in signal communication with the at least one x-ray detector and adapted to generate, from the acquired digital image data, a right-eye digital image and a left-eye digital image of the subject; and
a display adapted to display the right-eye and left-eye digital images of the subject to a viewer.

10. The imaging apparatus of claim 9 wherein the x-ray source array includes at least one carbon nanotube x-ray source.

11. The imaging apparatus of claim 9 wherein the display apparatus comprises a wearable viewing apparatus.

12. The imaging apparatus of claim 11 wherein the wearable viewing apparatus are shutter glasses and wherein the right-eye digital image and the left-eye digital image display over alternating time intervals.

13. The imaging apparatus of claim 11 wherein the wearable viewing apparatus has spectral filters and wherein the right-eye digital image and the left-eye digital image differ according to color spectra.

14. The imaging apparatus of claim 9 wherein the right-eye digital image has a first polarization state and the left-eye digital image has a second polarization state orthogonal to the first polarization state.

15. A method for obtaining stereoscopic radiography images of a subject, comprising:
providing a x-ray source array coupled to a support structure on a mobile transport frame, the x-ray source array having three or more x-ray sources, each x-ray source separately energizable to emit radiation toward a subject;
accepting at least one operator instruction specifying a first and a second x-ray source from the x-ray source array and indicating the specified first and second x-ray sources on a display;
obtaining digital image data from an x-ray detector adapted to acquire the digital image data according to radiation received from the specified first and a second x-ray source of the x-ray source array;
generating, from the acquired digital image data, a right-eye digital image and a left-eye digital image of the subject; and
displaying the right-eye and left-eye digital images of the subject.

16. The method of claim 15 wherein accepting the at least one operator instruction comprises accepting one or more signals from a viewing apparatus worn by an operator, wherein accepting the at least one operator instruction comprises observing operator movement, and wherein (1) the operator movement is hand movement, (2) the operator movement relates to body position, or (3) the operator movement is eye movement.
